(19) Europäisches Patentamt
European Patent Office
Office européen des brevets

(11) EP 4 578 439 A1

(12) # EUROPEAN PATENT APPLICATION
published in accordance with Art. 153(4) EPC

(43) Date of publication:
**02.07.2025 Bulletin 2025/27**

(21) Application number: **23857399.2**

(22) Date of filing: **24.08.2023**

(51) International Patent Classification (IPC):
***A61K 6/20*** *(2020.01)* ***A61K 6/30*** *(2020.01)*
***A61K 6/62*** *(2020.01)*

(52) Cooperative Patent Classification (CPC):
**A61K 6/20; A61K 6/30; A61K 6/62**

(86) International application number:
**PCT/JP2023/030400**

(87) International publication number:
**WO 2024/043291 (29.02.2024 Gazette 2024/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.08.2022 JP 2022134464**

(71) Applicant: **Kuraray Noritake Dental Inc.**
**Kurashiki-shi, Okayama 710-0801 (JP)**

(72) Inventors:
- **MATSUURA, Ryo**
  **Tainai-shi, Niigata 959-2653 (JP)**
- **MURAYAMA, Ryota**
  **Tainai-shi, Niigata 959-2653 (JP)**
- **NOJIRI, Yamato**
  **Tokyo 100-0004 (JP)**

(74) Representative: **D Young & Co LLP**
**3 Noble Street**
**London EC2V 7BQ (GB)**

(54) **DENTAL ADHESIVE COMPOSITION**

(57) The present invention provides a dental adhesive composition that enables the formation of a thin film as its cured product while achieving high mechanical strength over an extended period. The present invention relates to a dental adhesive composition comprising a monomer (A) having an acidic group, a monomer (B) having no acidic group, a polymerization initiator (C), water (D), a volatile organic solvent (E), and a filler (F), wherein the monomer (B) having no acidic group comprises a monomer (B-1) having three or more polymerizable groups per molecule, a hydrophobic monomer (B-2) having one or two polymerizable groups per molecule, and a hydrophilic monomer (B-3) having one or two polymerizable groups per molecule, the dental adhesive composition has a viscosity of less than 40 cps according to a B-type viscometer at 30°C, and the dental adhesive composition has a water absorbency of 4.0% or less in its cured product.

EP 4 578 439 A1

## Description

TECHNICAL FIELD

The present invention relates to dental adhesive compositions. More specifically, the present invention relates to a dental adhesive composition that enables the formation of a thin film as its cured product while achieving high mechanical strength over an extended period.

BACKGROUND ART

Adhesive materials find application in the restorative treatment of hard body tissues in moist bodies (for example, tooth structure, bones). Resin-based curable compositions containing components such as radical polymerizable monomers and polymerization initiators are frequently used as adhesive materials in such moist body applications.
Teeth that have lost their function due to issues such as decay or accidents are restored by, for example, fixing a dental prosthesis such as a crown restoration material, either metal or ceramic, known as an inlay or crown, to the affected teeth. An adhesive called dental resin cement is used to fix such crown restoration materials to the teeth.
In dental treatment, two restorative approaches exist: the direct restoration method involving the direct placement of a composite resin dental restoration material into the cavity, and the indirect restoration method whereby the crown restoration material is fixed to the tooth.
Typically, in the indirect restoration method, a temporary sealant is used to seal and protect the prepared tooth structure, or a temporary restoration is placed using a temporary bonding agent while the dental prosthesis is being prepared.
Given such situations, a technique has been proposed that is intended to enhance the adhesive properties of dental resin cement, improve the internal fit of the restoration, and protect the exposed dentin. In this technique, a dental adhesive is applied to the prepared cavity surface immediately after the cavity is formed and before taking an impression, in order to protect the exposed dentin and tooth pulp, and a temporary restoration material such as a temporary sealant or temporary bonding agent is placed to subsequently enhance the bonding of the dental resin cement to dentin (hereinafter, "resin-coating technique").
The dental adhesive used in resin-coating technique must create a thin coating layer to avoid affecting the shape of the prepared supporting tooth. To protect the exposed dentin, the dental adhesive is also required to possess high adhesive properties to dentin while exhibiting high mechanical strength as a coating layer. Concerning the shape of the prepared supporting tooth, the adhesive tends to collect around the margins of the supporting tooth. This tendency becomes more problematic especially when the adhesive has high viscosity. Reports have indicated that alterations in the shape of the supporting tooth coated by resin-coating technique may potentially affect the fit between dental prosthesis and the supporting tooth (Non Patent Literature 1).
The direct restoration method, commonly practiced with dental adhesives, does not require forming a thin adhesive layer. The formation of a thin film is a specific requirement in resin-coating technique.
Because the mechanical strength of the coating layer generally relies on its thickness, a thinner coating layer usually has low mechanical strength.
A type of dental adhesive that has become commonly used in recent years is a one-step adhesive system that uses a one-part dental adhesive (one-part bonding material) combining the functions of self-etching primer and bonding material. These one-part bonding materials typically contain monomers such as acidic monomers, hydrophilic monomers, and crosslinking monomers as monomer components. Typically, (meth)acrylate compounds are used as such monomer components.
To improve the mechanical strength of the cured product, Patent Literature 1 proposes a bonding material comprising: a polymerizable monomer with a non-conjugated carbon chain of four or more consecutive carbon atoms, two or more polymerizable groups, and two or more hydroxyl groups; a polymerizable monomer with one or more polymerizable groups and one or more aromatic rings; 2-hydroxyethyl methacrylate; 10-methacryloyloxydecamethylene phosphoric acid; water; ethanol; a polymerization accelerator; and a filler. Patent Literature 2 proposes a dental adhesive comprising: a polymerizable monomer component with an acidic group-containing polymerizable monomer; water; and a photopolymerization initiator composed of a diaryliodonium salt compound and a thioxanthone compound. Patent Literature 3 proposes a dental adhesive composition comprising: an acidic group-containing polymerizable monomer; a water-soluble (meth)acrylate polymerizable monomer; water; a curing agent; and a crosslinking polymerizable monomer in which at least three polymerizable groups, and a hydrocarbon group with at least six consecutive carbon atoms bonded in a linear or cyclic structure are present within its molecule.

CITATION LIST

Patent Literature

Patent Literature 1: JP 2008-260751 A
Patent Literature 2: JP 2007-223955 A
Patent Literature 3: JP 2005-179282 A

Non Patent Literature

Non Patent Literature 1: The 155th Meeting of The Japanese Society of Conservative Dentistry for Autumn 2021, O1

SUMMARY OF INVENTION

Technical Problem

Consideration could be given to using the dental bonding materials of Patent Literatures 1 to 3 for resin-coating purposes. However, upon investigation, the present inventors have found that Patent Literatures 1 and 3 involve high water absorbency in the cured product, suggesting that further improvement is needed to meet the properties required for resin-coating applications.
The composition of Patent Literature 2 was shown to lack sufficient mechanical strength for resin-coating applications.
As discussed above, there is still a need for improvement in the properties of dental adhesive compositions of related art if these were to be suitably used in resin-coating applications, which require forming a cured product thin enough to avoid affecting the fit between dental prosthesis and supporting tooth while enabling the protection of exposed dentin with excellent long-term mechanical strength.
It is accordingly an object of the present invention to provide a dental adhesive composition that enables the formation of a thin film as its cured product while achieving high mechanical strength over an extended period.

Solution to Problem

The present inventors conducted intensive studies, and found that the foregoing issues can be overcome with a dental adhesive composition that comprises specific components and satisfies specific parameters. The invention was completed after further studies.
Specifically, the present invention includes the following.

[1] A dental adhesive composition comprising a monomer (A) having an acidic group, a monomer (B) having no acidic group, a polymerization initiator (C), water (D), a volatile organic solvent (E), and a filler (F),

wherein the monomer (B) having no acidic group comprises a monomer (B-1) having three or more polymerizable groups per molecule, a hydrophobic monomer (B-2) having one or two polymerizable groups per molecule, and a hydrophilic monomer (B-3) having one or two polymerizable groups per molecule,
the dental adhesive composition has a viscosity of less than 40 cps according to a B-type viscometer at 30°C, and the dental adhesive composition has a water absorbency of 4.0% or less in its cured product.

[2] The dental adhesive composition according to [1], wherein the monomer (B-1) having three or more polymerizable groups per molecule comprises a hydrophobic compound.
[3] The dental adhesive composition according to [1] or [2], wherein the monomer (B-1) having three or more polymerizable groups per molecule comprises a compound having a urethane bond.
[4] The dental adhesive composition according to any one of [1] to [3], wherein the monomer (B-1) having three or more polymerizable groups per molecule comprises an aliphatic compound.
[5] The dental adhesive composition according to any one of [1] to [4], wherein the content of the monomer (B-1) having three or more polymerizable groups per molecule is 0.1 to 20 parts by mass in 100 parts by mass of all monomer components.
[6] The dental adhesive composition according to any one of [1] to [5], wherein the polymerizable groups in the monomer (B-1) having three or more polymerizable groups per molecule are (meth)acryloyloxy groups.
[7] The dental adhesive composition according to any one of [1] to [6], wherein the polymerization initiator (C)

comprises at least one selected from the group consisting of a (bis)acylphosphine oxide, an α-diketone, and a coumarin.

[8] The dental adhesive composition according to any one of [1] to [7], wherein the volatile organic solvent (E) comprises an alcohol solvent.

[9] The dental adhesive composition according to any one of [1] to [8], wherein at least one of the monomer (B-1) having three or more polymerizable groups per molecule, the hydrophobic monomer (B-2) having one or two polymerizable groups per molecule, and the hydrophilic monomer (B-3) having one or two polymerizable groups per molecule comprises a monomer having a (meth)acrylamide group.

[10] The dental adhesive composition according to [9], wherein the hydrophobic monomer (B-2) having one or two polymerizable groups per molecule comprises a monomer having a (meth)acrylamide group.

[11] The dental adhesive composition according to any one of [1] to [10], wherein the monomer (A) having an acidic group comprises a monomer having a phosphoric acid group and having one polymerizable group per molecule.

[12] The dental adhesive composition according to [11], wherein the content of the monomer having a phosphoric acid group and having one polymerizable group per molecule is 90 parts or more by mass in total 100 parts by mass of the monomer (A) having an acidic group contained in the dental adhesive composition.

[13] The dental adhesive composition according to any one of [1] to [12], wherein the monomer (B-1) having three or more polymerizable groups per molecule comprises a hydrophobic compound, and the polymerizable groups in the hydrophobic compound are all (meth)acryloyloxy groups.

[14] A dental bonding material according to any one of [1] to [13].

[15] A dental coating material according to any one of [1] to [13].

Advantageous Effects of Invention

According to the present invention, a dental adhesive composition can be provided that enables the formation of a thin film as its cured product while achieving high mechanical strength over an extended period. Because a dental adhesive composition of the present invention excels in durability, it can achieve the formation of a thin cured film and high long-term mechanical strength even when used for a prolonged period within the oral cavity.

A dental adhesive composition of the present invention also exhibits excellent bond strength to tooth structure over an extended period, enabling the protection of exposed dentin over a long period in resin-coating applications. This makes a dental adhesive composition of the present invention suitable for use in the indirect restoration method.

In a dental adhesive composition of the present invention, the cured product exhibits high mechanical strength over an extended period even when the coating thickness is 12 μm or less (preferably 10 μm or less, more preferably less than 7 μm) in resin-coating applications, where a thin coating is required. That is, the cured product can retain its mechanical strength over a long period even when the cured layer becomes thinner than the intended thickness as a result of, for example, thickness variation occurring in the layer due to the extent of air blowing when the layer is formed by evaporating the solvent components (for example, by blowing air). Consequently, there is no need for exact procedures during use, making the dental adhesive composition particularly advantageous in resin-coating applications.

A dental adhesive composition of the present invention also exhibits good cavity sealing properties with its excellent bond strength to tooth structure, making it also suitable for use as a dental adhesive composition in the common direct restoration method.

DESCRIPTION OF EMBODIMENTS

A dental adhesive composition of the present invention comprises a monomer (A) having an acidic group, a monomer (B) having no acidic group, a polymerization initiator (C), water (D), a volatile organic solvent (E), and a filler (F),

wherein the monomer (B) having no acidic group comprises a monomer (B-1) having three or more polymerizable groups per molecule, a hydrophobic monomer (B-2) having one or two polymerizable groups per molecule, and a hydrophilic monomer (B-3) having one or two polymerizable groups per molecule,

the dental adhesive composition has a viscosity of less than 40 cps according to a B-type viscometer at 30°C, and the dental adhesive composition has a water absorbency of 4.0% or less in its cured product.

In this specification, the term "(meth)acryl" is a collective term for methacryl and acryl, and the same applies to similar expressions, such as "(meth)acrylic acid" and "(meth)acrylonitrile".

In this specification, "monofunctional monomer" refers to monomers having one polymerizable group per molecule.

In this specification, the upper limits and lower limits of numeric ranges (for example, ranges of contents of components, ranges of values calculated from components, and ranges of physical properties) can be appropriately

combined. In this specification, various modifications can be made to the embodiments, such as by combining different embodiments as appropriate.

Generally, polyfunctional monomers, such as monomer (B-1) having three or more polymerizable groups per molecule, have the effect of enhancing mechanical strength in the composition. However, this leads to increased polymerization shrinkage as a result of an increase in curability. Additionally, increasing the content of polyfunctional monomers within the overall composition results in a relative decrease in the content of monofunctional monomers, which provide penetrability to the tooth structure. This results in reduced penetrability of the composition to the tooth structure, lowering the bond strength to the tooth structure.

The present inventors also discovered that the composition, in the absence of a hydrophobic monomer having one or two polymerizable group per molecule, cannot exhibit the sufficient level of hydrophobicity necessary for resin-coating applications when the content of polyfunctional monomers, such as monomer (B-1) having three or more polymerizable groups per molecule, is increased within the overall composition. As a result, the cured product cannot exhibit a sufficiently low water absorbency, making it impossible to achieve a thin coating layer and high mechanical strength in resin-coating applications.

Because of the difficulty in achieving both high mechanical strength and a thin coating layer in the resin-coating technique, it is even more challenging to provide a dental adhesive composition where the thin cured layer exhibits high mechanical strength over an extended period.

As discussed above, by simply increasing the content of polyfunctional monomers such as monomer (B-1) having three or more polymerizable groups per molecule, it is not possible to achieve long-term protection of the dentin exposed by cavity formation while reducing the thickness of the coating layer formed by the dental adhesive composition as its cured product to avoid affecting the shape of the prepared supporting tooth.

It remains unclear why a dental adhesive composition of the present invention enables the formation of a thin film as its cured product while achieving high mechanical strength over an extended period. However, the following speculation has been made.

In the present invention, the dental adhesive composition is configured by comprising appropriate types of monomer (A) having an acidic group, monomer (B) having no acidic group (including a monomer (B-1) having three or more polymerizable groups per molecule, a hydrophobic monomer (B-2) having one or two polymerizable groups per molecule, and a hydrophilic monomer (B-3) having one or two polymerizable groups per molecule), polymerization initiator (C), water (D), volatile organic solvent (E), and filler (F) in appropriate mixing ratios, leading to reduced viscosity in the dental adhesive composition through the integrated action of these components. This allows for the formation of a thin cured film. With its high curability and adequate hydrophobicity, the composition also appears to inhibit the generation of components that reduce the mechanical strength of the cured product, or the mechanism of action responsible for such reduction of mechanical strength, achieving high mechanical strength over an extended period.

A dental adhesive composition of the present invention has a viscosity of less than 40 cps at 30°C. The formation of a thin cured film is possible with the viscosity falling in this range, reducing the undesirable effect of the cured film on the fit between the dental prosthesis and supporting tooth. This viscosity range also allows for the formation of a considerably thin bond layer, and, by acting integrally with the predetermined water absorbency of the cured product, reduces a decrease in mechanical strength, despite the considerable thinness of the bond layer, leading to high long-term mechanical strength.

The viscosity can be adjusted by appropriately combining parameters such as the types, proportions, and viscosity of monomers, the type and content of filler, water content, and the type and content of volatile organic solvent.

The following provides descriptions of viscosity adjustment.

As a rule, monomers show increased viscosity as the number of polymerizable groups increases. The viscosity also increases when the monomer contains moieties that undergo intramolecular and intermolecular interactions. For example, the viscosity increases when the monomer contains functional groups capable of hydrogen bonding, such as hydroxyl or $NH_2$ groups. The monomer also shows increased viscosity when it is capable of forming ionic bonds, or contains aromatic rings. The type, proportion, and viscosity of monomer can be appropriately altered, taking into consideration properties such as the mechanical strength of the cured product, and adhesive properties to tooth structure.

In the present invention, the viscosity can be adjusted to fall within the desired range by using the monomer (B-1) having three or more polymerizable groups per molecule, marked by high viscosity, with monomers having one or two polymerizable groups per molecule (hydrophobic monomer (B-2) having one or two polymerizable groups per molecule, and hydrophilic monomer (B-3) having one or two polymerizable groups per molecule), along with filler (F) and solvent (water (D), volatile organic solvent (E)).

The filler tends to increase its effect of thickening the dental adhesive composition as the filler particle size decreases.

The thickening effect is affected by the presence or absence of surface treatment. For example, when using a filler with no surface treatment, the hydroxyl groups on the filler surface interact with other components of the dental adhesive composition, leading to a greater thickening effect.

The type and content of filler can be appropriately adjusted, taking into consideration properties such as the mechanical strength of the cured product, and adhesive properties to tooth structure.

The viscosity of the dental adhesive composition tends to decrease as the content of water and volatile organic solvent increases. When applying the dental adhesive composition to tooth structure, water and volatile organic solvent must be evaporated by air blowing because these components inhibit curing and weaken the mechanical strength of the cured product. It is therefore required to adjust the content of water and volatile organic solvent to appropriate levels. The water content, and the type and content of volatile organic solvent can be appropriately adjusted, taking into consideration properties such as the mechanical strength of the cured product, and adhesive properties to tooth structure.

In view of forming a thin cured film and its mechanical strength, the viscosity according to a B-type viscometer at 30°C is less than 40 cps, preferably 5 to 40 cps, more preferably 10 to 40 cps, even more preferably 15 to 35 cps, particularly preferably 20 to 30 cps. The method of viscosity measurement is as described in the EXAMPLES section below.

A dental adhesive composition of the present invention has a water absorbency of 4.0% or less in its cured product. With the water absorbency falling in this range, it is possible to form a thin coating layer in resin-coating applications while providing excellent mechanical strength. This water absorbency range also allows the cured product to exhibit superior adhesive properties to tooth structure and excellent mechanical strength, and, by acting integrally with the predetermined viscosity, provides not just excellent mechanical strength but superior durability in the cured product, allowing it retain these properties (adhesive properties to tooth structure, and mechanical strength) over an extended time period.

The water absorbency of the cured product can be adjusted according to the type and proportion of monomers, the type and amount of polymerization initiator, the type and amount of polymerization accelerator, the ratio of polymerization initiator and polymerization accelerator, the type, particle size, and content of filler, the amount of water, and the type and amount of volatile organic solvent. Adjusting the type and proportion of monomers is particularly effective in achieving the desired water absorbency. Making adjustments to other features, such as polymerization initiators, within the ranges specified in this specification can further facilitate achieving the desired water absorbency.

The descriptions below relate to adjustment of water absorbency.

Monomers with hydrophilic functional groups, such as hydroxyl groups, ether bonds, and amines, typically exhibit higher water absorbency, whereas monomers with no such functional groups show lower water absorbency.

Fillers with larger particle sizes have smaller specific surface areas, meaning less water adhesion to the surface and lower water absorbency. Smaller particles sizes increase the amount of water adhesion to the surface, leading to increased water absorbency.

Surface treatment of fillers increases the extent of surface hydrophobization, and reduces water absorbency. That is, the water absorbency can be reduced by increasing the extent of surface hydrophobization. One possible way of making the filler more hydrophobic is to use fillers having numerous surface hydroxyl groups (for example, such as inorganic fillers containing silica as the primary component) and promote dehydrocondensation reaction through surface treatment. Alternatively, fillers with inherently high hydrophobicity may be used to reduce water absorbency.

Hydrophilic monomers play a greater role in increasing water absorbency compared to fillers. Consequently, the water absorbency tends to decrease with a higher filler content and a lower content of hydrophilic monomers, whereas a lower filler content and a higher content of hydrophilic monomers tends to increase water absorbency. Typically, the water absorbency tends to increase more with organic fillers than with inorganic fillers or organic-inorganic composite fillers.

In view of the mechanical strength of the cured product, adhesive properties to tooth structure, and the durability of these properties, a dental adhesive composition of the present invention has a water absorbency of 4.0% or less, preferably 3.5% or less, more preferably 3.0% or less, even more preferably 2.5% or less, particularly preferably less than 2.3% in its cured product. A water absorbency higher than 4.0% may result in insufficient long-term mechanical strength in the cured product and insufficient adhesive properties to tooth structure.

The method of measurement of water absorbency is as described in the EXAMPLES section below.

In view of ease of forming a thin film and achieving high long-term mechanical strength in the resin-coating technique, it is preferable in a dental adhesive composition of the present invention that the cured product have a water absorbency of 7.5% or less, more preferably 7.0% or less, even more preferably 6.5% or less, particularly preferably less than 6.0% after immersion in distilled water at 37°C for 7 days.

The measurement method for the water absorbency of the cured product after immersion in distilled water at 37°C for 7 days is as described in the EXAMPLES section below.

The following describes each component used in a dental adhesive composition of the present invention.

<Monomer (A) Having an Acidic Group>

In view of adhesive properties to tooth structure, the dental adhesive composition requires a monomer (A) having an acidic group. The dental adhesive composition can exhibit excellent adhesive properties to tooth structure by

incorporating a monomer (A) having an acidic group. Preferred for use in the dental adhesive composition are radical polymerizable monomers.

Specific examples of radical polymerizable monomers in monomer (A) having an acidic group include (meth)acrylate monomers, (meth)acrylamide monomers, esters, vinyl esters, and vinyl ethers of acids such as α-cyanoacrylic acid, α-halogenated acrylic acid, crotonic acid, cinnamic acid, sorbic acid, maleic acid, and itaconic acid, as well as mono-N-vinyl derivatives and styrene derivatives. In view of curability, preferred among these are (meth)acrylate monomers and (meth)acrylamide monomers in which the polymerizable groups are (meth)acryloyloxy groups or (meth)acrylamide groups.

In view of superior adhesive properties to tooth structure (particularly dentin) and ease of inhibiting a viscosity increase in the dental adhesive composition when combined with other monomers, the monomer (A) having an acidic group is preferably a monomer having one or two polymerizable groups per molecule (monofunctional monomer or bifunctional monomer), more preferably a monomer having one polymerizable group per molecule.

Examples of the monomer (A) having an acidic group used in the present invention include monomers having at least one acidic group such as a phosphoric acid group, a pyrophosphoric acid group, a thiophosphoric acid group, a phosphonic acid group, a carboxylic acid group, and a sulfonic acid group.

The monomer (A) having an acidic group may be used alone, or two or more thereof may be used in appropriate combinations. Specific examples of the monomer (A) having an acidic group are as follows.

Examples of monomers having a phosphoric acid group include monofunctional monomers such as 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, 2-(meth)acryloyloxyethylphenyl hydrogen phosphate, 2-(meth)acryloyloxyethyl-(2-bromoethyl)hydrogen phosphate, 2-methacryloyloxyethyl-(4-methoxyphenyl)hydrogen phosphate, and 2-methacryloyloxypropyl-(4-methoxyphenyl)hydrogen phosphate; and bifunctional monomers such as bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, and 1,3-glycerol di(meth)acrylate phosphate (also known as 1,3-di(meth)acryloyloxypropyl dihydrogen phosphate), as well as acid chlorides, alkali metal salts, and amine salts of these monofunctional monomers and bifunctional monomers. Preferred are monomers having a C6 to C12 alkylene group and a divalent phosphoric acid group.

A certain preferred embodiment is, for example, a dental adhesive composition in which the monomer (A) having an acidic group comprises a monomer having a phosphoric acid group and having one polymerizable group per molecule.

Another certain preferred embodiment is, for example, a dental adhesive composition in which the content of the monomer having a phosphoric acid group and having one polymerizable group per molecule is 90 parts or more by mass in total 100 parts by mass of the monomer (A) having an acidic group contained in the dental adhesive composition.

**In** such an embodiment, the content of the monomer having a phosphoric acid group and having one polymerizable group per molecule is preferably 92 parts or more by mass, more preferably 95 parts or more by mass, even more preferably 98 parts or more by mass, particularly preferably 100 parts by mass in total 100 parts by mass of the monomer (A) having an acidic group.

Yet another certain preferred embodiment is, for example, a dental adhesive composition in which the monomer (A) having an acidic group comprises a monomer having a C6 to C12 alkylene group and a divalent phosphoric acid group.

In any of these preferred embodiments, the polymerizable group of the monomer is preferably a (meth)acryloyloxy group.

Examples of monomers having a pyrophosphoric acid group include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, and amine salts of these.

Examples of monomers having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyicosyl dihydrogen thiophosphate, and acid chlorides, alkali metal salts, ammonium salts of these.

Examples of monomers having a phosphonic acid group include 2-(meth)acryloyloxyethylphenylphosphonate,

5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl-3-phosphonoacetate, 10-(meth)acryloyloxydecyl-3-phosphonoacetate, and acid chlorides, alkali metal salts, and ammonium salts of these.

Examples of monomers having a carboxylic acid group include monofunctional (meth)acrylic acid esters having one carboxyl group or acid anhydride group thereof per molecule, and monofunctional (meth)acrylic acid esters having a plurality of carboxyl groups or acid anhydride groups thereof per molecule.

Examples of monofunctional monomers having one carboxyl group or acid anhydride group thereof per molecule include (meth)acrylic acid, N-(meth)acryloylglycine, N-(meth)acryloylaspartic acid, 2-(meth)acryloyloxyethyl hydrogen succinate, 2-(meth)acryloyloxyethyl hydrogen phthalate, 2-(meth)acryloyloxyethyl hydrogen malate, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, and compounds derived by converting the carboxyl group of these compounds into an acid anhydride group.

Examples of monofunctional monomers having a plurality of carboxyl groups or acid anhydride groups thereof per molecule include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyltrimellitate, 4-(meth)acryloyloxyethyltrimellitic anhydride, 4-(meth)acryloyloxybutyltrimellitate, 4-(meth)acryloyloxyhexyltrimellitate, 4-(meth)acryloyloxydecyltrimellitate, 2-(meth)acryloyloxyethyl-3'-(meth)acryloyloxy-2'-(3,4-dicarboxybenzoyloxy)propylsuccin ate, 6-(meth)acryloyloxyethylnaphthalene-1,2,6-tricarboxylic anhydride, 6-(meth)acryloyloxyethylnaphthalene-2,3,6-tricarboxylic anhydride, 4-(meth)acryloyloxyethylcarbonylpropionoyl-1,8-naphthalic anhydride, and 4-(meth)acryloyloxyethylnaphthalene-1,8-tricarboxylic anhydride.

Examples of monomers having a sulfonic acid group include 2-sulfoethyl (meth)acrylate.

In view of the dental adhesive composition exhibiting favorable bond strength to the tooth structure, preferred among these examples of monomer (A) having an acidic group are monomers having a phosphoric acid group, or monomers having a carboxylic acid group, more preferably 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, 4-(meth)acryloyloxyethyltrimellitate anhydride, 4-(meth)acryloyloxyethyltrimellitate, 11-(meth)acryloyloxyundecane-1,1-dicarboxylic acid, and a mixture of 2-methacryloyloxyethyl dihydrogen phosphate and bis(2-methacryloyloxyethyl)hydrogen phosphate, even more preferably, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, and 20-(meth)acryloyloxyicosyl dihydrogen phosphate. In view of ease of inhibiting a viscosity increase in the dental adhesive composition and a balance between bond strength to tooth structure and curability, 10-(meth)acryloyloxydecyl dihydrogen phosphate is particularly preferred.

In view of ease of inhibiting a viscosity increase in the dental adhesive composition when combined with other monomers and from the viewpoint of adhesive properties to tooth structure, the content of the monomer (A) having an acidic group in the dental adhesive composition is preferably 1 to 50 mass%, more preferably 1 to 30 mass%, even more preferably 3 to 20 mass% in the total mass of the dental adhesive composition.

<Monomer (B) Having no Acidic Group>

Examples of the monomer (B) having no acidic group in the present invention include a monomer (B-1) having three or more polymerizable groups per molecule, a hydrophobic monomer (B-2) having one or two polymerizable groups per molecule, and a hydrophilic monomer (B-3) having one or two polymerizable groups per molecule.

· Monomer (B-1) Having Three or More Polymerizable Groups per Molecule

The monomer (B-1) having three or more polymerizable groups per molecule (hereinafter, also referred to simply as "polyfunctional monomer (B-1)") is used to achieve both a thin cured film and high mechanical strength in a dental adhesive composition of the present invention.

Examples of the polymerizable groups include vinyl groups, (meth)acryloyloxy groups, and (meth)acrylamide groups. In view of ease of radical polymerization, preferred are (meth)acryloyloxy groups and/or (meth)acrylamide groups. In view of adhesive properties to dentin, (meth)acrylamide groups are preferred.

The polyfunctional monomer (B-1) may be used alone, or two or more thereof may be used in combination.

The polyfunctional monomer (B-1), which has three or more polymerizable groups, may have four or more polymerizable groups. There is no particular restriction on the maximum number of polymerizable groups, and it may be 10 or less, or 6 or less.

In view of enabling a sufficient increase in hydrophobicity and a further reduction in the water absorbency of the cured product, the polyfunctional monomer (B-1) preferably comprises a hydrophobic compound.

The hydrophobic compound is preferably a compound having a solubility of less than 10 mass% in 25°C water. The hydrophobicity of the hydrophobic compound can be adjusted with hydrophilic groups. Examples of hydrophilic groups include at least one selected from the group consisting of a hydroxyl group, an oxymethylene group, an oxyethylene group, an oxypropylene group, and an amide group.

The hydrophobic compound is preferably a compound with no hydrophilic group, more preferably a compound with no hydroxyl group.

A certain preferred embodiment is, for example, a dental adhesive composition in which the hydrophobic compound comprises a compound having one or fewer hydroxyl groups.

Another preferred embodiment is, for example, a dental adhesive composition in which the polymerizable groups in the hydrophobic compound are all (meth)acryloyloxy groups.

In view of mechanical strength, the polyfunctional monomer (B-1) preferably comprises a compound having a urethane bond. In view of viscosity, the polyfunctional monomer (B-1) preferably comprises an aliphatic compound. In view of mechanical strength, it is even more preferable for the polyfunctional monomer (B-1) to comprise an aliphatic compound having a urethane bond.

Examples of the polyfunctional monomer (B-1) include trimethylolpropane tri(meth) acrylate, ethoxylated trimethylolpropane tri(meth) acrylate, trimethylolethane tri(meth) acrylate, trimethylolmethane tri(meth) acrylate, pentaerythritol tri(meth) acrylate, pentaerythritol tetra(meth)acrylate, ethoxylated pentaerythritol tetra(meth)acrylate, dipentaerythritol penta(meth)acrylate, dipentaerythritol poly(meth)acrylate, ethoxylated dipentaerythritol poly(meth)acrylate, ditrimethylolpropane tetramethacrylate, ethoxylated glycerin triacrylate, tris-2-(meth)acryloyloxyethyl)isocyanurate, N,N-(2,2,4-trimethylhexamethylene)bis[2-(aminocarboxy)propane-1,3-diol]tetra(meth)acr ylate, 1,7-diacryloyloxy-2,2,6,6-tetra(meth)acryloyloxymethyl-4-oxaheptane, N,N',N"-triacryloyldiethylenetriamine, N, N', N", N‴-tetraacryloyltriethylenetetramine, and compounds represented by the formulae (1) to (3) below. In view of mechanical strength, preferred among these are trimethylolpropane tri(meth) acrylate, N,N-(2,2,4-trimethylhexamethylene)bis[2-(aminocarboxy)propane-1,3-diol]tetramethacryl ate, ditrimethylolpropane tetramethacrylate, and N,N',N",N‴-tetraacryloyltriethylenetetramine.

[Chem. 1]

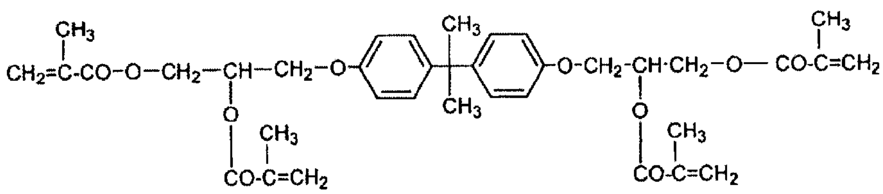

(1)

[Chem. 2]

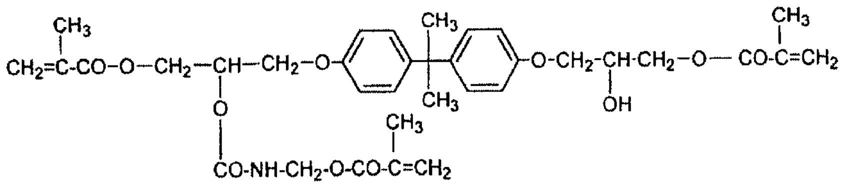

(2)

[Chem. 3]

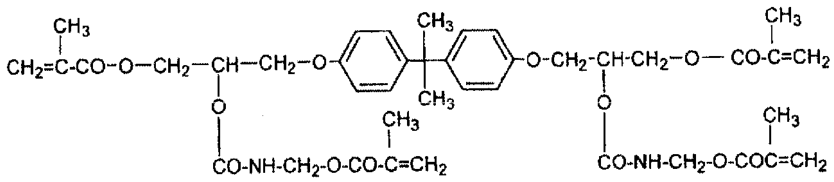

(3)

In view of long-term mechanical strength, viscosity, and adhesive properties to tooth structure, the content of the monomer (B-1) having three or more polymerizable groups per molecule in a dental adhesive composition of the present invention is preferably 0.1 to 20 mass%, more preferably 0.5 to 15 mass%, even more preferably 1 to 10 mass%, particularly preferably 2 to 8 mass% in the total mass of the dental adhesive composition.

When the content of monomer (B-1) having three or more polymerizable groups per molecule is 20 mass% or less, it is possible to sufficiently increase hydrophobicity when combined with other components such as the hydrophobic monomer (B-2) having one or two polymerizable groups per molecule. This facilitates achieving desired water absorbency in the cured product, and, together with the predetermined viscosity, allows for the formation of a thin coating layer and excellent long-term mechanical strength in the resin-coating technique.

In certain embodiments, the content of monomer (B-1) having three or more polymerizable groups per molecule is preferably 0.1 to 20 parts by mass, more preferably 0.5 to 15 parts by mass, even more preferably 1 to 10 parts by mass, particularly preferably 2 to 8 parts by mass in 100 parts by mass of all monomer components in the dental adhesive composition.

When the content of monomer (B-1) having three or more polymerizable groups per molecule is 20 parts or less by mass in 100 parts by mass of all monomer components in the dental adhesive composition, it is possible to sufficiently increase hydrophobicity when combined with other components such as the hydrophobic monomer (B-2) having one or two polymerizable groups per molecule. This facilitates achieving desired water absorbency in the cured product, and, together with the predetermined viscosity, allows for the formation of a thin coating layer and excellent long-term mechanical strength in the resin-coating technique.

· Hydrophobic Monomer (B-2)

The hydrophobic monomer (B-2) having one or two polymerizable groups per molecule (hereinafter, also referred to simply as "hydrophobic monomer (B-2)") refers to a monomer that has no acidic group, and that has a solubility of less than 10 mass% in 25°C water.

The hydrophobic monomer (B-2) is preferably one having a solubility of less than 5 mass%, more preferably less than 1 mass% in 25°C water.

The hydrophobic monomer (B-2) can reduce water absorbency while improving properties such as the mechanical strength of the cured product of the dental adhesive composition, and durability (bond strength to tooth structure, and mechanical strength).

Preferably, the hydrophobic monomer (B-2) is a radical polymerizable monomer having no acidic group and having a polymerizable group(s).

Examples of the polymerizable groups in the hydrophobic monomer (B-2) include vinyl groups, (meth)acryloyloxy groups, and (meth)acrylamide groups. In view of ease of radical polymerization, preferred are (meth)acryloyloxy groups and/or (meth)acrylamide groups. In view of adhesive properties to dentin, (meth)acrylamide groups are preferred.

In view of water absorbency, long-term mechanical strength, and adhesive properties to dentin, it is more preferable to incorporate hydrophobic monomer (B-2) as a compound having a (meth)acrylamide group and a (meth) acryloyloxy group, even more preferably a compound having an acrylamide group and a methacryloyloxy group.

The hydrophobic monomer (B-2) may be used alone, or two or more thereof may be used in combination.

Examples of the hydrophobic monomer (B-2) include monofunctional monomers having one polymerizable group per molecule, and bifunctional monomers having two polymerizable groups per molecule.

Examples of bifunctional monomers having two polymerizable groups per molecule include aromatic bifunctional hydrophobic monomers, and aliphatic bifunctional hydrophobic monomers.

Examples of monofunctional monomers as hydrophobic monomers (B-2) include: aliphatic monofunctional (meth)acrylate monomers such as n-stearyl methacrylate; aliphatic monofunctional (meth)acrylate monomers having an ether bond, such as butoxydiethylene glycol methacrylate, methoxypolyethylene glycol methacrylate (with an average of nine moles of oxyethylene group added); alicyclic monofunctional (meth)acrylate monomers such as cyclohexyl methacrylate, isobornyl methacrylate, and dicyclopentanyl methacrylate; monofunctional (meth)acrylate monomers having an aromatic ring group, such as 2-phenoxyethyl methacrylate, and phenoxybenzyl methacrylate; and monofunctional (meth)acrylate monomers having a heterocyclic ring group (for example, a cyclic ether group), such as tetrahydrofurfuryl methacrylate.

The monofunctional (meth)acrylate monomers having an aromatic ring group are preferably those with one or two phenyl groups.

The monofunctional (meth)acrylate monomers having a heterocyclic ring group are preferably those with one or two heterocyclic ring groups (for example, cyclic ether groups).

In view of mechanical strength and viscosity, preferred among these monofunctional monomers are tetrahydrofurfuryl methacrylate (commonly known as THF-MA), benzyl methacrylate (commonly known as BEMA), phenoxybenzyl methacrylate (commonly known as POB-MA), and 2-phenoxyethyl methacrylate (commonly known as PEMA).

Examples of the aromatic bifunctional hydrophobic monomers include 2,2-bis((meth)acryloyloxyphenyl)propane, 2,2-bis[4-(3-(meth)acryloyloxy-2-hydroxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane.

In view of adhesive properties to tooth structure and mechanical strength, preferred among these are 2,2-bis [4-(2-hydroxy-3-methacryloyloxypropoxy)phenyl]propane (commonly known as Bis-GMA), 2,2-bis(4-(meth)acryloyloxyethoxyphenyl)propane, 2,2-bis(4-methacryloyloxypolyethoxyphenyl)propane (with an average of 2.6 moles of ethoxy group added, commonly known as D-2.6E), 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, and 2,2-bis(4-(meth)acryloyloxypentaethoxyphenyl)propane.

Examples of the aliphatic bifunctional hydrophobic monomers include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 2,2,4-trimethylhexamethylenebis(2-carbamoyloxyethyl)di(meth)acrylate, N-methacryloyloxyethylacrylamide (commonly known as MAEA), N-methacryloyloxypropylacrylamide, N-methacryloyloxybutylacrylamide, N-(1-ethyl-(2-methacryloyloxy)ethyl)acrylamide, and N-(2-(2-methacryloyloxyethoxy)ethyl)acrylamide.

In view of mechanical strength and handling properties, preferred among these are triethylene glycol diacrylate, triethylene glycol dimethacrylate (commonly known as 3G), neopentyl glycol dimethacrylate (commonly known as NPG), 2,2,4-trimethylhexamethylenebis(2-carbamoyloxyethyl)dimethacrylate (commonly known as UDMA), and 1,10-decanediol dimethacrylate (commonly known as DD).

In view of polymerization shrinkage stress, UDMA and DD are preferred.

In view of adhesive properties to tooth structure, particularly dentin, N-methacryloyloxypropylacrylamide (MAEA) is preferred.

In view of water absorbency, mechanical strength, solubility in water, and handling properties, preferred for use as hydrophobic monomer (B-2) are aromatic bifunctional hydrophobic monomers and aliphatic bifunctional hydrophobic monomers.

Preferred as aromatic bifunctional monomers are Bis-GMA and D-2.6E.

Preferred as aliphatic bifunctional monomers are 3G, neopentyl glycol di(meth)acrylate, UDMA, DD, and MAEA.

The hydrophobic monomer (B-2) in the dental adhesive composition is preferably 5 to 60 mass%, more preferably 10 to 50 mass%, even more preferably 12 to 45 mass%, particularly preferably 15 to 40 mass% in the total mass of the dental adhesive composition.

When the content of hydrophobic monomer (B-2) is at or below these upper limits, it is easier to inhibit a decrease in the wettability of the dental adhesive composition to the tooth structure, and a resulting reduction of adhesive strength. When the content of hydrophobic monomer (B-2) is at or above the foregoing lower limits, it is easier to achieve the desired mechanical strength and water absorbency in the cured product.

· Hydrophilic Monomer (B-3)

The hydrophilic monomer (B-3) having one or two polymerizable groups per molecule (hereinafter, also referred to simply as "hydrophilic monomer (B-3)") refers to a monomer that has no acidic group, and that has a solubility of 10 mass% or more in 25°C water.

The hydrophilic monomer (B-3) is preferably one having a solubility of 30 mass% or more in 25°C water, more preferably one that can dissolve in water in any proportions at 25°C.

The hydrophilic monomer (B-3) can improve the bond strength to tooth structure by improving the wettability of the dental adhesive composition to tooth structure, and the penetrability of the dental adhesive composition to tooth structure (enamel/dentin).

Preferably, the hydrophilic monomer (B-3) is a radical polymerizable monomer having no acidic group and having a polymerizable group(s).

Examples of the polymerizable groups in the hydrophilic monomer (B-3) include vinyl groups, (meth)acryloyloxy groups, and (meth)acrylamide groups. In view of ease of radical polymerization, preferred are (meth)acryloyloxy groups and/or (meth)acrylamide groups. In view of adhesive properties to dentin, (meth)acrylamide groups are preferred.

The hydrophilic monomer (B-3) may be incorporated alone, or two or more thereof may be used in combination.

Preferred as hydrophilic monomer (B-3) are those having a hydrophilic group, such as a hydroxyl group, an oxymethylene group, an oxyethylene group, an oxypropylene group, and an amide group.

Examples of the hydrophilic monomer (B-3) include monofunctional monomers having one polymerizable group per molecule, and bifunctional monomers having two polymerizable groups per molecule.

Examples of monofunctional monomers as hydrophilic monomers (B-3) include hydrophilic monofunctional (meth)acrylate monomers such as 2-hydroxyethyl (meth)acrylate, 3-hydroxypropyl (meth)acrylate, 2-hydroxypropyl (meth)acrylate, 1,3-dihydroxypropyl (meth)acrylate, 2,3-dihydroxypropyl (meth)acrylate, and 2-((meth)acryloyloxy)ethyltrimethylammonium chloride; and hydrophilic monofunctional (meth)acrylamide monomers such as N-methylol(meth) acrylamide, N-hydroxyethyl(meth)acrylamide, N,N-bis(2-hydroxyethyl)(meth)acrylamide, N-methoxymethyl(meth)acrylamide, N-ethoxymethyl(meth)acrylamide, diacetone(meth)acrylamide, 4-(meth)acryloylmorpholine, N-trihydroxymethyl-N-methyl(meth)acrylamide, N,N-dimethylacrylamide, and N,N-diethylacrylamide.

Examples of the bifunctional monomers include hydrophilic bifunctional (meth)acrylate monomers such as polyethylene glycol di(meth)acrylate (with an average of nine or more moles of oxyethylene group added), 1,2-bis(3-(meth)acryloyloxy-2-hydroxypropoxy)ethane, and 3-(meth)acryloyloxy-2-hydroxypropyl (meth)acrylate.

In view of adhesive properties to tooth structure, preferred among these hydrophilic monomers (B-3) are 2-hydroxyethyl (meth)acrylate, 2,3-dihydroxypropyl (meth)acrylate, and hydrophilic monofunctional (meth)acrylamide monomers, more preferably 2-hydroxyethyl (meth)acrylate, N,N-dimethylacrylamide, and N,N-diethylacrylamide.

In view of adhesive properties to tooth structure, the content of the hydrophilic monomer (B-3) in the dental adhesive composition is preferably 5 to 60 mass%, more preferably 7 to 50 mass%, even more preferably 10 to 45 mass%, particularly preferably 13 to 40 mass% in the total mass of the dental adhesive composition.

When the content of the hydrophilic monomer (B-3) in the dental adhesive composition is at or above these lower limits, it is easier to produce a sufficient bond strength improving effect. When the content of the hydrophilic monomer (B-3) in the dental adhesive composition is at or below the foregoing upper limits, it is easier to achieve the desired mechanical strength and water absorbency in the cured product.

In view of achieving both a thin cured film and long-term mechanical strength, the content of the monomer (B) having no acidic group in the dental adhesive composition is preferably 20 to 95 mass%, more preferably 25 to 90 mass%, even more preferably 30 to 85 mass% in the total mass of the dental adhesive composition.

In view of adhesive properties to tooth structure and the water absorbency of the cured product, the mass ratio of hydrophobic monomer (B-2) to hydrophilic monomer (B-3) is preferably 1:0.1 to 1:3, more preferably 1:0.2 to 1:2, even more preferably 1:0.3 to 1:1, particularly preferably 1:0.4 to 1:0.9.

In view of achieving adequate hydrophobicity in the dental adhesive composition and facilitating adjustment of water absorbency in the cured product to further enhance the long-term mechanical strength of the cured product together with other features such as the predetermined viscosity and fillers, it is preferable in a certain preferred embodiment that the content of hydrophobic monomer (B-2) be higher than that of polyfunctional monomer (B-1). From this viewpoint, the mass ratio of polyfunctional monomer (B-1) to hydrophobic monomer (B-2) is preferably 1:1.1 to 1:500, more preferably 1:1.2 to 1:300, even more preferably 1:1.3 to 1:200.

In view of more easily achieving even higher bond strength to tooth structure over an extended period and even higher long-term mechanical strength in the cured product together with other features such as the predetermined viscosity and fillers, it is preferable in another certain preferred embodiment that the content of hydrophilic monomer (B-3) be higher than that of polyfunctional monomer (B-1). From this viewpoint, the mass ratio of polyfunctional monomer (B-1) to hydrophilic monomer (B-3) is preferably 1:1.1 to 1:150, more preferably 1:1.2 to 1:100, even more preferably 1:1.3 to 1:80.

Yet another certain preferred embodiment is, for example, a dental adhesive composition in which at least one of the monomer (B-1) having three or more polymerizable groups per molecule, the hydrophobic monomer (B-2) having one or two polymerizable groups per molecule, and the hydrophilic monomer (B-3) having one or two polymerizable groups per molecule comprises a monomer having a (meth)acrylamide group.

In such a preferred embodiment, it is preferable that the hydrophobic monomer (B-2) and/or hydrophilic monomer (B-3) comprise a monomer having a (meth)acrylamide group. More preferably, the hydrophobic monomer (B-2) comprises a monomer having a (meth)acrylamide group.

For example, when the hydrophobic monomer (B-2) comprises a monomer having a (meth)acrylamide group, the hydrophobic monomer (B-2) may comprise a monomer having an acrylamide group.

<Polymerization Initiator (C)>

The polymerization initiator (C) is an essential component for curing the monomers. The polymerization initiator (C) can reduce water absorbency while improving properties such as the mechanical strength of the cured product of the dental adhesive composition, and durability (bond strength to tooth structure, and mechanical strength). The polymerization initiator (C) may be a photopolymerization initiator (C-1) or chemical polymerization initiator (C-2). The polymerization initiator (C) may be incorporated alone, or two or more thereof may be used in combination.

· Photopolymerization Initiator (C-1)

In view of adhesive properties to tooth structure, curability, and mechanical strength, the dental adhesive composition preferably comprises a photopolymerization initiator (C-1).

Preferably, the photopolymerization initiator (C-1) is a water-insoluble photopolymerization initiator having a solubility of less than 10 g/L in 25°C water (hereinafter, also referred to simply as "water-insoluble photopolymerization initiator"), or a water-soluble photopolymerization initiator having a solubility of 10 g/L or more in 25°C water (hereinafter, also referred to simply as "water-soluble photopolymerization initiator"). In view of curability and mechanical strength, it is preferable to comprise a water-insoluble photopolymerization initiator. In certain embodiments, in view of adhesive properties to tooth structure, a water-soluble photopolymerization initiator may be used with a water-insoluble photopolymerization initiator.

The photopolymerization initiator (C-1) may be a known photopolymerization initiator. The photopolymerization initiator (C-1) may be incorporated alone, or two or more thereof may be incorporated in combination.

Examples of the water-insoluble photopolymerization initiator include (bis)acylphosphine oxides, thioxanthones, ketals, $\alpha$-diketones, coumarins, anthraquinones, benzoin alkyl ether compounds, and $\alpha$-aminoketone compounds.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, and benzoyl di(2,6-dimethylphenyl)phosphonate. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide.

Examples of the thioxanthones include thioxanthone, and 2-chlorothioxanthen-9-one.

Examples of the ketals include benzyl dimethyl ketal, and benzyl diethyl ketal.

Examples of the $\alpha$-diketones include diacetyl, benzyl, dl-camphorquinone, 2,3-pentadione, 2,3-octadione, 9,10-phenanthrenequinone, 4,4'-oxybenzyl, and acenaphthenequinone. Particularly preferred is dl-camphorquinone for its maximum absorption wavelength occurring in the visible light region.

Examples of the coumarins include compounds mentioned in JP H09-3109 A and JP H10-245525 A, including, for example, 3,3'-carbonylbis(7-diethylaminocoumarin), 3-(4-methoxybenzoyl)coumarin, 3-thienoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzoimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-3-[4-(dimethylamino)phenyl]-1-oxo-2-propenyl]-2,3,6,7-tetrahydro-1,1,7,7-tetramethy l-1H,5H,11H-[1]benzopyrrano[6,7,8-ij]quinolizin-11-one, and 10-(2-benzothiazolyl)-2,3,6,7-tetrahydro-1,1,7,7-tetramethyl-1H,5H,11H-[1]benzopyrrano [6,7,8-ij]quinolizin-11-one.

Particularly preferred among these coumarins are 3,3'-carbonylbis(7-diethylaminocoumarin) and 3,3'-carbonylbis(7-dibutylaminocoumarin).

Examples of the anthraquinones include anthraquinone, 1-chloroanthraquinone, 2-chloroanthraquinone, 1-bromoanthraquinone, 1,2-benzanthraquinone, 1-methylanthraquinone, 2-ethylanthraquinone, and 1-hydroxyanthraquinone.

Examples of the benzoin alkyl ether compounds include benzoin methyl ether, benzoin ethyl ether, benzoin isopropyl ether, and benzoin isobutyl ether.

Examples of the α-aminoketone compounds include 2-methyl-1-[4-(methylthio)phenyl]-2-morpholinopropan-1-one.

Among these water-insoluble photopolymerization initiators, it is preferable to comprise at least one selected from the group consisting of a (bis)acylphosphine oxide, an α-diketone, and a coumarin. In this way, a dental adhesive composition can be obtained that has excellent photocurability both in the visible light region and the near ultraviolet region, and that shows sufficient photocurability regardless of whether the light source used is a halogen lamp, a light emitting diode (LED), or a xenon lamp.

The water-soluble photopolymerization initiator can improve the polymerization curability at the interface with hydrophilic tooth surfaces, resulting in high bond strength. The water-soluble photopolymerization initiator has a solubility in 25°C water of 10 g/L or more, preferably 15 g/L or more, more preferably 20 g/L or more, even more preferably 25 g/L or more. By having a solubility of 10 g/L or more in 25°C water, the water-soluble photopolymerization initiator can sufficiently dissolve in water within the tooth structure at the bonding interface, and more easily exhibits the polymerization promoting effect.

Examples of the water-soluble photopolymerization initiator include water-soluble thioxanthones; water-soluble acylphosphine oxides; and α-hydroxyalkylacetophenones, for example, such as compounds having a (poly)ethylene glycol chain introduced into the hydroxyl group(s) of 1-[4-(2-hydroxyethoxy)-phenyl]-2-hydroxy-2-methyl-1-propan-1-one, compounds having a (poly)ethylene glycol chain introduced into the hydroxyl group and/or phenyl group of 1-hydroxycyclohexyl phenyl ketone, compounds having $-OCH_2COO^-Na^+$ introduced into the phenyl group of 1-hydroxycyclohexyl phenyl ketone, compounds having a (poly)ethylene glycol chain introduced into the hydroxyl group and/or phenyl group of 2-hydroxy-2-methyl-1-phenylpropan-1-one, and compounds having $-OCH_2COO^-Na^+$ introduced into the phenyl group of 2-hydroxy-2-methyl-1-phenylpropan-1-one. Other examples of the water-soluble photopolymerization initiator include quaternary ammonium compounds prepared by quaternization of the amino group of α-aminoalkylphenones, such as 2-methyl-1-[4-(methylthio)phenyl]-2-morpholinopropan-1-one, and 2-benzyl-2-(dimethylamino)-1-[(4-morpholino)phenyl]-1-butanone.

The water-soluble thioxanthones may be any of, for example, 2-hydroxy-3-(9-oxo-9H-thioxanthen-4-yloxy)-N,N,N-trimethyl-1-propaneaminium chloride, 2-hydroxy-3-(1-methyl-9-oxo-9H-thioxanthen-4-yloxy)-N,N,N-trimethyl-1-propaneaminiu m chloride, 2-hydroxy-3-(9-oxo-9H-thioxanthen-2-yloxy)-N,N,N-trimethyl-1-propaneaminium chloride, 2-hydroxy-3-(3,4-dimethyl-9-oxo-9H-thioxanthen-2-yloxy)-N,N,N-trimethyl-1-propaneami nium chloride, 2-hydroxy-3-(3,4-dimethyl-9H-thioxanthen-2-yloxy)-N,N,N-trimethyl-1-propaneaminium chloride, and 2-hydroxy-3-(1,3,4-trimethyl-9-oxo-9H-thioxanthen-2-yloxy)-N,N,N-trimethyl-1-propanea minium chloride.

Preferred among these water-soluble acylphosphine oxides are sodium phenyl(2,4,6-trimethylbenzoyl)phosphinate, lithium phenyl(2,4,6-trimethylbenzoyl)phosphinate, sodium bis(2,4,6-trimethylbenzoyl)phosphinate, and lithium bis(2,4,6-trimethylbenzoyl)phosphinate.

The content of photopolymerization initiator (C-1) is not particularly limited. However, in view of considerations such as the curability of the dental adhesive composition obtained, the content of photopolymerization initiator (C-1) is preferably 0.01 to 10 mass%, more preferably 0.05 to 7 mass%, even more preferably 0.1 to 5 mass% in the total mass of the dental adhesive composition.

· Chemical Polymerization Initiator (C-2)

In view of enabling chemical polymerization and improving cavity sealing properties in areas with no access to light, the dental adhesive composition may additionally comprise a chemical polymerization initiator (C-2).

The chemical polymerization initiator (C-2) may be used alone, or two or more thereof may be used in combination.

Preferably, the chemical polymerization initiator (C-2) is an organic peroxide.

The organic peroxides used as chemical polymerization initiator (C-2) are not particularly limited, and known organic peroxides may be used.

Typical examples of such organic peroxides include ketone peroxides, hydroperoxides, diacyl peroxides, dialkyl peroxides, peroxyketals, peroxyesters, and peroxydicarbonates. Specific examples of these organic peroxides include those mentioned in WO2008/087977.

The content of chemical polymerization initiator (C-2) is not particularly limited. However, in view of considerations such as the curability of the dental adhesive composition obtained, the content of chemical polymerization initiator (C-2) is preferably 0.01 to 10 mass%, more preferably 0.05 to 7 mass%, even more preferably 0.1 to 5 mass% in the total mass of the dental adhesive composition.

<Water (D)>

In the present invention, water (D) promotes the demineralizing effect of the monomer (A) having an acidic group. It is necessary that water (D) be essentially free of impurities that adversely affect the adhesive properties. Preferred for use is distilled water or ion-exchange water. The bond strength to tooth structure may decrease if the content of water (D) is either too high or too low. The content of water (D) is preferably 1 to 50 mass%, more preferably 5 to 30 mass%, even more preferably 10 to 20 mass% in the total mass of the dental adhesive composition.

<Volatile Organic Solvent (E)>

In view of bond strength to tooth structure, coatability, the formation of a thin cured film, and the prevention of phase separation of components, a volatile organic solvent (E) needs to be incorporated in a dental adhesive composition of the present invention. The volatile organic solvent (E) helps adjust the viscosity of the dental adhesive composition, and, together with other components, enables the formation of a thin cured film. In view of application to methods such as the resin-coating technique, the organic solvent incorporated in a dental adhesive composition of the present invention must be volatile.

The volatile organic solvent (E) may be used alone, or two or more thereof may be used in combination.

In view of compatibility with other components of the dental adhesive composition, the volatile organic solvent (E) is preferably a water-soluble volatile organic solvent. The volatile organic solvent is preferably one with a boiling point of 150°C or less under ordinary pressure, more preferably one with a boiling point of 100°C or less under ordinary pressure.

The water-soluble volatile organic solvent is preferably one with a solubility of 5 mass% or more in 25°C water, more preferably one with a solubility of 30 mass% or more in 25°C water, even more preferably one that can dissolve in water in any proportions.

Specific examples of volatile organic solvent (E) include alcohol solvents such as ethanol, methanol, 1-propanol, and isopropyl alcohol; ketone solvents such as acetone, and methyl ethyl ketone; and ether solvents such as 1,2-dimethoxyethane, 1,2-diethoxyethane, and tetrahydrofuran. Alcohol solvents are preferred in view of providing excellent compatibility, and thus preventing phase separation of components and achieving even superior storage stability.

The bond strength may decrease if the content of volatile organic solvent (E) is too high. In view of achieving the desired viscosity as a dental adhesive composition, the content of volatile organic solvent (E) is preferably 1 to 70 mass%, more preferably 5 to 50 mass%, even more preferably 10 to 30 mass% in the total mass of the dental adhesive composition.

<Filler (F)>

The dental adhesive composition comprises a filler (F) to adjust curability, the water absorbency of the cured product, the mechanical strength of the thin film formed upon curing, and handling properties. Examples of the filler (F) include inorganic fillers, organic-inorganic composite fillers, and organic fillers.

The filler (F) may be used alone, or two or more thereof may be used in combination.

Examples of the inorganic fillers include various types of glass, such as fused silica, quartz, soda-lime-silica glass, E glass, C glass, borosilicate glass (PYREX® glass), strontium·borosilicate glass, fluoroaluminosilicate glass, aluminosilicate glass, calcium fluoroaluminosilicate glass, strontium fluoroaluminosilicate glass, strontium calcium fluoroaluminosilicate glass, and barium glass (barium silicate glass, barium boroaluminosilicate glass, barium fluoroaluminosilicate glass). Other examples include lanthanum glass-ceramics, various ceramics, alumina, composite oxides (for example, silica-containing composite oxides such as silica-titania, silica-titania-barium oxide, silica-zirconia, silica-alumina, and silica-alumina-zirconia), ytterbium oxide, silica-coated ytterbium fluoride, diatomaceous earth, kaolin, clay mineral (such as montmorillonite), activated earth, synthetic zeolite, mica, calcium fluoride, ytterbium fluoride, yttrium fluoride, calcium phosphate, barium sulfate, zirconium oxide, titanium oxide, and hydroxyapatite. These may be used alone, or two or more thereof may be used in combination.

In view of storage stability, preferred among these are fused silica and silica-zirconia, more preferably fused silica.

In certain embodiments, the material of inorganic fillers preferably comprises various types of glass (those containing silica as the main component (a silica content of 5 mass% or more, preferably 10 mass% or more), and, optionally, oxides of elements such as heavy metals, boron, and aluminum).

In view of considerations such as the handling properties and mechanical strength of the dental adhesive composition obtained, the inorganic fillers have an average particle diameter of preferably 0.001 to 50 μm, more preferably

0.001 to 1 μm, even more preferably 0.005 to 0.1 μm, particularly preferably 0.005 to 0.05 μm. In the present invention, the average particle diameter of inorganic fillers means the average particle diameter before surface treatment when inorganic fillers are subjected to surface treatment, as will be described. A certain preferred embodiment is, for example, a dental adhesive composition in which the filler (F) is an inorganic filler.

The inorganic fillers may be commercially available products. Examples of such commercially available products include silica such as Aerosil® 90, Aerosil® 130, Aerosil® 150, Aerosil® 200, Aerosil® 255, Aerosil® 300, Aerosil® 380, Aerosil® OX50, and Aerosil® R972 (all manufactured by Nippon Aerosil Co., Ltd.), GM27884, 8235 (manufactured by Schott), barium glass such as E-3000 (product code; manufactured by Esstech), strontium·borosilicate glass (E-4000, manufactured by ESSTECH), lanthanum glass-ceramic (GM31684, manufactured by Schott), and fluoroaluminosilicate glass (GM35429, G018-091, and G018-117, manufactured by Schott). In view of considerations such as adhesive strength and coatability, preferred as filler (F) are silica with small primary particle diameters, such as Aerosil® 90, Aerosil® 130, Aerosil® 150, Aerosil® 200, Aerosil® 255, Aerosil® 300, Aerosil® 380, Aerosil® OX50, and Aerosil® R972.

The inorganic fillers may be amorphous or crystalline, or a combination of these. However, the inorganic fillers are preferably at least partially amorphous. The shape of inorganic fillers is not particularly limited, and may be appropriately selected for use.

In order to confine the viscosity of the dental adhesive composition within the desired range by adjusting the mechanical strength and flowability of the dental adhesive composition, it is preferable to use the inorganic fillers after a surface treatment with a known surface treatment agent such as a silane coupling agent. For example, the hydroxyl groups present on the surface of inorganic fillers may be surface treated with a surface treatment to obtain inorganic fillers having its hydroxyl groups surface treated. Surface treatment can reduce the thickening effect due to the addition of fillers.

Examples of the surface treatment agent include silane coupling agents such as vinyltrimethoxysilane, vinyltriethoxysilane, vinyltrichlorosilane, vinyltri(β-methoxyethoxy)silane, 3-methacryloyloxypropyltrimethoxysilane, 8-methacryloyloxyoctyltrimethoxysilane, 11-methacryloyloxyundecyltrimethoxysilane, γ-glycidoxypropyltrimethoxysilane, γ-mercaptopropyltrimethoxysilane, and γ-aminopropyltriethoxysilane. Preferred are vinyltrimethoxysilane, 3-methacryloyloxypropyltrimethoxysilane, 8-methacryloyloxyoctyltrimethoxysilane, 11-methacryloyloxyundecyltrimethoxysilane, and γ-aminopropyltriethoxysilane.

The surface treatment can be carried out using known methods, with no particular restrictions.

Examples of such methods include a method that adds the surface treatment agent by spraying it while vigorously stirring the inorganic filler, a method that disperses or dissolves the inorganic filler and surface treatment agent in a suitable solvent, and removes the solvent, and a method in which the alkoxy group of the surface treatment agent is transformed into a silanol group by hydrolysis with an acid catalyst in an aqueous solution, and the silanol group is allowed to adhere to the inorganic filler surface in the aqueous solution before removal of water. In all of these methods, the reaction between the surface of inorganic filler and the surface treatment agent can proceed to completion to enable surface treatment by applying heat in a temperature range of typically 50 to 150°C. The amount of surface treatment is not particularly limited. For example, the amount of surface treatment agent may be 0.1 to 40 parts by mass relative to 100 parts by mass of inorganic filler before surface treatment.

The organic-inorganic composite fillers are fillers obtained by adding a monomer to the inorganic filler, polymerizing the mixture in paste form, and pulverizing the resulting material. The organic-inorganic composite filler refers to a filler comprising the inorganic filler and a polymer of a monomer.

Examples of the monomer include the monomer (A) having an acidic group, and the monomer (B) having no acidic group. Preferred is the hydrophobic monomer (B-2).

The organic-inorganic composite filler may be, for example, a filler obtained by mixing Bis-GMA, 3G, and a surface-treated silica filler, and pulverizing the mixture after polymerization. The shape of the organic-inorganic composite filler is not particularly limited, and the particle diameter of fillers may be appropriately selected for use.

The organic-inorganic composite fillers may be used alone, or two or more thereof may be used in combination.

In view of mechanical strength, the organic-inorganic composite fillers are preferably surface-treated. Examples of surface treatment agents and the preferred varieties of surface treatment agents are no different from those described in conjunction with inorganic fillers.

In view of considerations such as the handling properties and mechanical strength of the dental adhesive composition obtained, the organic-inorganic composite fillers have an average particle diameter of preferably 0.001 to 50 μm, more preferably 0.001 to 20 μm, even more preferably 0.005 to 15 μm.

Examples of the materials of the organic fillers include polymethylmethacrylate, polyethylmethacrylate, a methyl methacrylate-ethyl methacrylate copolymer, crosslinked polymethylmethacrylate, crosslinked polyethylmethacrylate, polyamides, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used as a mixture.

The shape of organic fillers is not particularly limited, and the particle diameter of fillers may be appropriately selected for use.

In view of considerations such as the handling properties and mechanical strength of the dental adhesive composition obtained, the average particle diameter of the organic fillers is preferably 0.001 to 50 μm, more preferably 0.001 to 20 μm, even more preferably 0.005 to 15 μm.

In this specification, the average particle diameter of fillers can be determined by a laser diffraction scattering method or by observing particles with an electron microscope. Specifically, a laser diffraction scattering method is more convenient for the measurement of particles having a particle size of 0.1 μm or more, whereas electron microscopy is a more convenient method of particle size measurement for ultrafine particles of less than 0.1 μm. Here, 0.1 μm is a measured value by a laser diffraction scattering method. In particles such as agglomerated particles formed by primary particles, there are average particle diameters for primary particles and secondary particles. In such cases, the average particle diameter of fillers refers to the average particle diameter of the larger secondary particles.

As a specific example of a laser diffraction scattering method, the particle size may be measured by volume using, for example, a laser diffraction particle size distribution analyzer (SALD-2300, manufactured by Shimadzu Corporation) with a 0.2% sodium hexametaphosphate aqueous solution used as dispersion medium.

In electron microscopy, for example, particles may be photographed with an electron microscope (Model S-4000, manufactured by Hitachi), and the size of particles (at least 200 particles) observed in a unit field of the captured image may be measured using image-analyzing particle-size-distribution measurement software (Mac-View, manufactured by Mountech Co., Ltd.). Here, the particle diameter is determined as an arithmetic mean value of the maximum and minimum lengths of particles, and the average particle diameter is calculated from the number of particles and the particle diameter.

The refractive index of filler (F) is not particularly limited, and may be, for example, 1.35 to 2.00. In view of the transparency of the cured product, the refractive index of filler (F) is preferably 1.40 to 1.70. The refractive index of filler (F) can be controlled by, for example, adjusting the types and/or proportions of the constituents of the filler (F). In this specification, the refractive index of the subject (for example, filler (F), colorants) can be measured using an Abbe refractometer.

The content of filler (F) is not particularly limited. However, in view of achieving the desired viscosity as a dental adhesive composition and forming a thin cured film, and from the viewpoint of the water absorbency of the cured product, handling properties, and the mechanical strength of the cured product, the content of filler (F) is preferably 0.1 to 30 mass%, more preferably 0.5 to 20 mass%, even more preferably 1 to 10 mass% in the total mass of the dental adhesive composition.

When the content of filler (F) is confined within these ranges, the dental adhesive composition, together with other features, can have a viscosity within the desired range, reducing a decrease in mechanical strength despite the considerable thinness of the bond layer formed as the cured product, facilitating high mechanical strength over an extended period.

<Polymerization Accelerator (G)>

In view of the mechanical strength of the cured product, the dental adhesive composition preferably comprises a polymerization accelerator (G).

Examples of the polymerization accelerator (G) used in the present invention include amines, sulfinic acid and salts thereof, borate compounds, barbituric acid compounds, triazine compounds, copper compounds, tin compounds, vanadium compounds, halogen compounds, aldehydes, thiol compounds, sulfites, bisulfites, and thiourea compounds.

The polymerization accelerator (G) may be incorporated alone, or two or more thereof may be used in combination.

The amines used as polymerization accelerator (G) can be categorized into aliphatic amines and aromatic amines. Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In view of the curability and storage stability of the dental adhesive composition, preferred for use are tertiary aliphatic amines, more preferably N-methyldiethanolamine and triethanolamine.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, propyl 4-(N,N-dimethylamino)benzoate, n-butoxyethyl

4-(N,N-dimethylamino)benzoate, 2-(methacryloyloxy)ethyl 4-(N,N-dimethylamino)benzoate, 4-(N,N-dimethylamino) benzophenone, and butyl 4-(N,N-dimethylamino)benzoate. In view of the ability to impart excellent curability to the dental adhesive composition, it is preferable to use at least one selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone.

Specific examples of sulfinic acid and salts thereof, borate compounds, barbituric acid compounds, triazine compounds, copper compounds, tin compounds, vanadium compounds, halogen compounds, aldehydes, thiol compounds, sulfites, bisulfites, and thiourea compounds include those mentioned in WO2008/087977.

The content of the polymerization accelerator (G) used in the present invention is not particularly limited. However, in view of the curability and mechanical strength of the dental adhesive composition obtained, the content of polymerization accelerator (G) ranges preferably from 0.01 to 10 mass%, more preferably from 0.05 to 7 mass%, even more preferably from 0.1 to 5 mass% in the total mass of the dental adhesive composition.

The dental adhesive composition may additionally comprise a fluorine-ion releasing substance. By containing a fluorine-ion releasing substance, the dental adhesive composition produced can impart acid resistance to tooth structure. Examples of the fluorine-ion releasing substance include fluorine-ion releasing polymers such as copolymers of methyl methacrylate and methacrylic acid fluoride; and metal fluorides such as sodium fluoride, potassium fluoride, sodium monofluorophosphate, lithium fluoride, and ytterbium fluoride. The fluorine-ion releasing substance may be incorporated alone, or two or more thereof may be incorporated in combination.

The content of the fluorine-ion releasing substance is preferably 0.0001 to 10 mass%, more preferably 0.005 to 5 mass%, even more preferably 0.01 mass% to 2 mass% in the total mass of the dental adhesive composition.

The dental adhesive composition may also comprise a known additive, provided that such additives do not cause a performance drop. Examples of additives that may be contained in the dental adhesive composition include polymerization inhibitors, antioxidants, colorants (pigments, dyes), ultraviolet absorbers, fluorescent agents, silane coupling agents, and thickeners.

The additives may be used alone, or two or more thereof may be used in combination.

In view of adjusting storage stability and curability, a dental adhesive composition of the present invention preferably comprises a polymerization inhibitor. Examples of the polymerization inhibitor include hydroquinone, hydroquinone monomethyl ether, dibutylhydroquinone, dibutylhydroquinone monomethyl ether, t-butylcatechol, 2-t-butyl-4,6-dimethylphenol, 2,6-di-t-butylphenol, and 3,5-di-t-butyl-4-hydroxytoluene. These may be used alone, or two or more thereof may be used in combination.

The content of the polymerization inhibitor is preferably 0.001 to 1.0 mass% in the total mass of the dental adhesive composition.

In view of photostability against ambient light from light sources such as fluorescent lamps and LEDs, and reducing discoloration of the cured product, a dental adhesive composition of the present invention preferably comprises an ultraviolet absorber. Examples of the ultraviolet absorber include benzotriazole compounds such as 2-(2-hydroxyphenyl)benzotriazole, 2-(2-hydroxy-5-methylphenyl)benzotriazole, 2-(2-hydroxy-5-ethylphenyl)benzotriazole, 2-(2-hydroxy-5-propylphenyl)benzotriazole, 2-(2-hydroxy-5-tert-octylphenyl)benzotriazole, and 2-(3-tert-butyl-2-hydroxy-5-methylphenyl)-5-chloro-2H-benzotriazole (Tinuvin 326); and benzoimidazole compounds. Preferred is Tinuvin 326. These may be used alone, or two or more thereof may be used in combination.

In view of adhesive properties to tooth structure and long-term mechanical strength, a dental adhesive composition of the present invention preferably comprises a silane coupling agent, separately from the surface treatment agents for fillers. Examples of the silane coupling agent include vinyltrimethoxysilane, vinyltriethoxysilane, vinyltrichlorosilane, vinyltri(β-methoxyethoxy)silane, 3-methacryloyloxypropyltrimethoxysilane, 8-methacryloyloxyoctyltrimethoxysilane, 11-methacryloyloxyundecyltrimethoxysilane, γ-glycidoxypropyltrimethoxysilane, γ-mercaptopropyltrimethoxysilane, γ-aminopropyltriethoxysilane, 3-(meth)acryloyloxypropylmethyldimethoxysilane, 3-(meth)acryloyloxypropylmethyldiethoxysilane, and 3-(meth)acryloyloxypropyldimethylmonomethoxysilane. In view of adhesive properties, preferred are vinyltrimethoxysilane, 3-methacryloyloxypropyltrimethoxysilane, 8-methacryloyloxyoctyltrimethoxysilane, 11-methacryloyloxyundecyltrimethoxysilane, γ-aminopropyltriethoxysilane, 3-(meth)acryloyloxypropylmethyldimethoxysilane, 3-(meth)acryloyloxypropylmethyldiethoxysilane, 3-(meth)acryloyloxypropyldimethylmonomethoxysilane, and hydrolysates of these.

The silane coupling agent may be used alone, or two or more thereof may be used in combination.

The content of the silane coupling agent is preferably 0.001 to 30 mass%, more preferably 0.05 to 20 mass%, even more preferably 0.1 mass% to 10 mass% in the total mass of the dental adhesive composition.

Examples of the preferred composition ratios of the dental adhesive composition are as follows.

The dental adhesive composition comprises preferably 1 to 50 mass% of monomer (A) having an acidic group, 0.1 to 20 mass% of monomer (B-1) having three or more polymerizable groups per molecule, 5 to 60 mass% of hydrophobic monomer (B-2), 5 to 60 mass% of hydrophilic monomer (B-3), 0.01 to 10 mass% of photopolymerization initiator (C-1), 1 to 50 mass% of water (D), 1 to 70 mass% of volatile organic solvent (E), 0.1 to 30 mass% of filler (F), and

0.01 to 10 mass% of polymerization accelerator (G), more preferably 1 to 30 mass% of monomer (A) having an acidic group, 0.5 to 15 mass% of monomer (B-1) having three or more polymerizable groups per molecule, 10 to 50 mass% of hydrophobic monomer (B-2), 7 to 50 mass% of hydrophilic monomer (B-3), 0.05 to 7 mass% of photopolymerization initiator (C-1), 5 to 30 mass% of water (D), 5 to 50 mass% of volatile organic solvent (E), 0.5 to 20 mass% of filler (F), and 0.05 to 7 mass% of polymerization accelerator (G) in the total mass of the dental adhesive composition.

A dental adhesive composition comprising a monomer (A) having an acidic group, a monomer (B) having no acidic group, a polymerization initiator (C), water (D), a volatile organic solvent (E), a filler (F) along with optional components can be produced with ease using methods known to a person skilled in the art.

A dental adhesive composition of the present invention can be suitably used in dental bonding material and dental coating material applications, particularly, dental bonding material and dental coating material applications for resin-coating.

A dental adhesive composition of the present invention has a viscosity of less than 40 cps according to a B-type viscometer at 30°C, enabling the formation of a thin coating layer in a temperature range (for example, 35°C or less) simulating use at room temperature in dental clinics.

A dental adhesive composition of the present invention may be used with materials such as dental etchants and dental primers. These dental etchants and dental primers may be used individually or in combination.

There are no specific limitations on dental etchants, dental primers, and other such materials, and commercially available products may be used.

<Specific Application Method and Procedure>

The dental adhesive composition is applied to the surface of the tooth structure being treated. After application, water (D) and volatile organic solvent (E) are removed from the dental adhesive composition by blowing air that has been adjusted to an appropriate pressure that does not displace the dental adhesive composition from the application site.

In the case where the dental adhesive composition contains a photopolymerization initiator (C-1), the dental adhesive composition is cured by exposing it to light with a dental photoirradiator.

When containing a chemical polymerization initiator (C-2), the dental adhesive composition may be left undisturbed until it completely cures. In certain cases, this may be followed by filling a separately prepared dental composite resin.

The dental composite resin is not particularly limited, and may be a commercially available product.

When using the dental adhesive composition with other components such as dental etchants and dental primers, these materials are used before the application of the dental adhesive composition.

EXAMPLES

The following describes the present invention in greater detail using Examples and Comparative Examples. However, the present invention is not limited to the following. In the following, "part(s)" means "part(s) by mass", unless otherwise specifically stated.

The components of the dental adhesive compositions of Examples and Comparative Examples are presented below, along with the abbreviations.

[Monomer (A) Having Acidic Group]

MDP: 10-methacryloyloxydecyl dihydrogen phosphate
GDMP: 1,3-glycerol dimethacrylate phosphate
4-META: 4-methacryloyloxyethyl trimellitate anhydride

[Monomer (B) Having no Acidic Group]

· Monomer (B-1) having three or more polymerizable groups per molecule

TMPMA: trimethylolpropane tri(meth)acrylate (a solubility of less than 10 mass% in 25°C water)
U4TH:
N,N-(2,2,4-trimethylhexamethylene)bis[2-(aminocarboxy)propane-1,3-diol]tetramethacryl ate (a solubility of less than 10 mass% in 25°C water)
TAC4: N,N',N'',N'''-tetraacryloyltriethylenetetramine (a solubility of 10 mass% or more in 25°C water)
DTMP: ditrimethylolpropane tetramethacrylate (a solubility of less than 10 mass% in 25°C water)
Bis-4: Compound of formula (3) (a solubility of less than 10 mass% in 25°C water)

[Chem. 4]

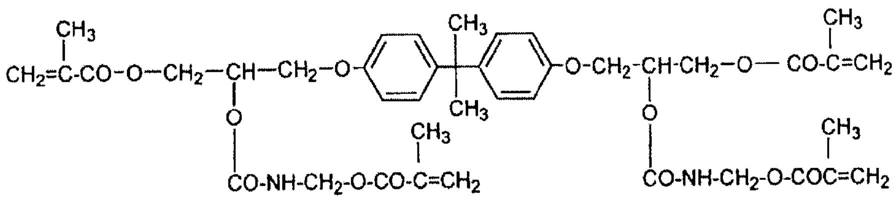

(3)

· Hydrophobic monomer (B-2)

Bis-GMA: 2,2-bis[4-(2-hydroxy-3-methacryloyloxypropoxy)phenyl]propane
3G: triethylene glycol dimethacrylate
MAEA: N-methacryloyloxyethylacrylamide
UDMA: [2,2,4-trimethylhexamethylenebis(2-carbamoyloxyethyl)]dimethacrylate
NPG: neopentyl glycol dimethacrylate

· Hydrophilic monomer (B-3)

DEAA: N,N-diethylacrylamide
HEMA: 2-hydroxyethyl methacrylate
#801: 1,2-bis(3-methacryloyloxy-2-hydroxypropoxy)ethane

[Photopolymerization Initiator (C-1)]

CQ: dl-camphorquinone
BAPO: bis(2,4,6-trimethylbenzoyl)phenylphosphine oxide
TMDPO: 2,4,6-trimethylbenzoyldiphenylphosphine oxide

[Water (D)]
Purified water
[Volatile Organic Solvent (E)]

Ethanol
Acetone

[Filler (F)]
R972: hydrophobic fumed silica manufactured by Nippon Aerosil Co., Ltd.; ultrafine particulate silica Aerosil® R972, average particle diameter: 16 nm (silica), refractive index: 1.46
[Polymerization Accelerator (G)]

DABE: ethyl 4-(N,N-dimethylamino)benzoate
DEPT: N,N-bis(2-hydroxyethyl)-p-toluidine

[Others]

BHT: 3,5-di-t-butyl-4-hydroxytoluene (polymerization inhibitor)
KBM-503: 3-methacryloyloxypropyltrimethoxysilane (silane coupling agent)
Sodium fluoride

<Examples 1 to 21 and Comparative Examples 1 to 7>

[Preparation of Dental Adhesive Composition]

From the raw materials listed in Tables 1 to 3, the components excluding the filler, water, and volatile organic solvent were initially mixed together. This was followed by the addition of the filler, and, subsequently, water and volatile organic solvent to obtain a mixture. The mixture was then stirred at ordinary temperature (23°C) in a dark environment to

create a homogenous mixture, and this mixture was ultrasonically degassed to prepare a liquid dental adhesive composition.

Test Example 1: Water Absorbency

The liquid dental adhesive composition prepared was transferred into a screw cap bottle, and air was continuously applied to evaporate water and volatile organic solvent from the dental adhesive composition. The composition was then poured into a SUS mold (15 mm in diameter, 1.0 mm thick), and pressed with glass slides after placing polyester films on both the top and bottom surfaces (15 mm in diameter). With the glass slides pressing the composition, the composition was cured by exposing it to light through the glass slides using a dental visible-light irradiator (PenCure 2000, manufactured by J. Morita Corp.) in standard mode, with light applied from the top and bottom for 10 seconds at five locations on each side (totaling 50 seconds of exposure per side), resulting in a cured product. Ten samples of the cured product were prepared for each Example and Comparative Example. The cured product was then removed from the mold, and its mass, denoted as $m_1$, was measured for each sample after removing burrs.

Subsequently, the samples were immersed in distilled water (30 mL) in a container. In this state, five of the samples were left for 24 hours inside a thermostatic chamber that had been set to 37°C, whereas the remaining five samples were left in a thermostatic chamber at 37°C for 7 days. After removing the samples from the distilled water, the mass was measured and recorded as $m_2$ after removing any remaining water on the sample.

The water absorbency was calculated using the formula below. Measurements were taken for each sample of the cured product, and the average was calculated (n = 5).

$$W_{sp} (\%) = (m_2 - m_1)/m_1 (g/g) \times 100$$

$m_1$: Mass of the sample after being immersed in water for 24 hours (μg)
$m_2$: Mass of the sample before immersion in water (μg)

In view of the mechanical strength of the cured product, adhesive properties to the tooth structure, and durability, the water absorbency of the cured product of the dental adhesive composition of the present invention after 24 hours of immersion at 37°C (hereinafter, also referred to as "initial water absorbency") is 4.0% or less, preferably 3.5% or less, more preferably 3.0% or less, even more preferably 2.5% or less, particularly preferably less than 2.3%. A water absorbency greater than 4.0% may result in insufficient mechanical strength in the cured product and insufficient adhesive properties to the tooth structure.

In view of the long-term mechanical strength of the cured product, adhesive properties to the tooth structure, and durability, the water absorbency of the cured product of the dental adhesive composition of the present invention after 7 days of immersion at 37°C (hereinafter, also referred to as "enduring water absorbency") is preferably 7.5% or less, more preferably 7.0% or less, even more preferably 6.5% or less, particularly preferably less than 6.0%.

Test Example 2: Shear Bond Strength (Enamel, Dentin)

A dental adhesive composition of the present invention exhibits excellent bond strength to tooth structure over an extended period, and provides long-lasting protection for exposed dentin in resin-coating applications, making it suitable for use in the indirect restoration method. A dental adhesive composition of the present invention also exhibits good cavity sealing properties with its excellent bond strength to tooth structure, making it also suitable for use as a dental adhesive composition in the common direct restoration method.

Although this test example is a system intended for the direct restoration method, it remains essentially the same, in terms of adhesive properties to tooth structure, even when applied to resin-coating applications, allowing it to be also used to assess the bond strength to tooth structure in resin-coating applications.

The test was conducted in compliance with ISO 29022:2013, specifically as follows.

The labial surfaces of bovine teeth were polished with #80 silicon carbide paper (manufactured by Nihon Kenshi Co., Ltd.) under running water to obtain samples with an exposed flat enamel surface and samples with an exposed flat dentin surface.

First, the shear bond strength to enamel was measured using samples with an exposed flat enamel surface, as follows.

A mold with 15 holes (15-hole mold, manufactured by Ultradent Products Inc., 35 mm in diameter × 25 mm in height) was separately prepared and a tape was attached to the bottom of the mold. The bovine tooth sample was then secured onto the tape.

Thereafter, a resin for dental impression trays (Tray Resin II manufactured by Shofu Inc. under this trade name)

was filled into the mold, and left to stand for about 30 minutes to cure. This resulted in a composite of bovine tooth and cured resin. This composite was then removed from the mold to obtain a sample.

The composite had the bovine tooth exposed at the top surface of the cured resin. The top surface of the sample was polished under running water to a size large enough to provide a bonding surface (a diameter of 2.38 mm or more), using #600 silicon carbide paper (manufactured by Nihon Kenshi Co., Ltd.), and the bonding surface was ultrasonically washed with water for 5 minutes.

Subsequently, the dental adhesive composition (adhesive) prepared in each Example and Comparative Example was applied to the bovine tooth surface. After 3 seconds of standing, a mild air blow was applied to evaporate water and volatile organic solvent. The adhesive was then cured by exposing it to light for 10 seconds, using a dental visible-light irradiator (PenCure 2000, manufactured by J. Morita Corp.) in standard mode.

A separately prepared CR filling mold (Bonding Mold Insert, manufactured by Ultradent Products Inc.; Ø = 2.38 mm) was installed on a dedicated instrument (Bonding Clamp, manufactured by Ultradent Products Inc.). The sample was then secured by lowering the CR filling mold, allowing the CR filling mold installed on the dedicated instrument to contact the bonding surface of the sample.

Thereafter, Clearfil® AP-X (manufactured by Kuraray Noritake Dental Inc.) was filled into the hole of the CR filling mold to form a thin layer of no greater than 1 mm. After filling another portion of Clearfil® AP-X into the CR filling mold (to about 2/3 of the mold, or about 2 mm thick), it was irradiated with light for 20 seconds to cure, using a dental visible-light irradiator (PenCure 2000, manufactured by J. Morita Corp.) in standard mode. The sample was then removed from the CR filling mold, and used as an adhesion test sample. A total of twenty adhesion test samples were prepared for each Example and Comparative Example.

The adhesion test sample was immersed in distilled water inside a container, and left to stand in this state for 24 hours in a thermostatic chamber with the chamber temperature set to 37°C. Ten of the samples were immediately measured for shear bond strength after being taken out of distilled water. (hereinafter, also referred to as "initial shear bond strength"). The average values of the measurements are indicated by "Initial" in the Tables. For the measurement of bond durability, the remaining ten samples were additionally subjected to 4,000 cycles of thermal cycling involving alternating immersion in 4°C cold water and 60°C hot water for 1 minute each, and the shear bond strength was measured (hereinafter, also referred to as "enduring shear bond strength"). The average values of the measurements are indicated by "Enduring" in the Tables.

For the measurement of bond strength (shear bond strength), the adhesion test sample was installed on a dedicated holder (Test Base Clamp, manufactured by Ultradent Products Inc.), and measurements were taken using a dedicated jig (Crosshead Assembly, manufactured by Ultradent Products Inc.) and a universal testing machine (manufactured by Shimadzu Corporation) with the crosshead speed set at 1 mm/min. The average values are presented in the Tables (n = 10).

The shear bond strength to dentin was measured in the same manner as for enamel. The average values are presented in the Tables (n = 10).

The preferred initial shear bond strength to enamel in the dental adhesive compositions of the present invention is 15 MPa or more, more preferably 20 MPa or more, even more preferably 25 MPa or more.

The preferred enduring shear bond strength to enamel is 10 MPa or more, more preferably 15 MPa or more, even more preferably 20 MPa or more.

The preferred initial shear bond strength to dentin in the dental adhesive compositions of the present invention is 15 MPa or more, more preferably 20 MPa or more, even more preferably 25 MPa or more.

The preferred enduring shear bond strength to dentin is 10 MPa or more, more preferably 15 MPa or more, even more preferably 20 MPa or more.

Test Example 3: Tensile Strength Test

An appropriate amount of each dental adhesive composition was added into a glass beaker, and, while stirring the composition, air was continuously applied to evaporate the water and volatile organic solvent contained in the dental adhesive composition. After placing a polyester film on a glass slide, a Teflon® mold (1 mm × 1 mm × 10 mm) was set on this film.

Following the evaporation of water and volatile organic solvent, the dental adhesive composition was poured into the Teflon® mold. After placing a polyester film and a glass slide on the composition, the composition was cured by exposing it to light for 10 seconds from both sides using a dental visible-light irradiator (PenCure 2000, manufactured by J. Morita Corp.) in normal mode

The resulting cured product was then removed from the Teflon® mold, and unwanted burrs were removed. Ten samples were prepared in each Example and Comparative Example. These samples were immersed in distilled water inside a container, and left to stand in this state for 24 hours in a thermostatic chamber with the chamber temperature set to 37°C. The samples were then measured for tensile strength using a universal testing machine (Autograph AG-I 100kN,

manufactured by Shimadzu Corporation) at a crosshead speed of 1.0 mm/min (n = 5), and the average value was calculated. The average values of the measurements are indicated by "Initial" in the Tables.

The remaining five samples, immersed in distilled water inside a container, were left to stand in this state for 7 days in a thermostatic chamber at 37°C. The samples were then measured for tensile strength using a universal testing machine (Autograph AG-I 100kN, manufactured by Shimadzu Corporation) at a crosshead speed of 1.0 mm/min (n = 5), and the average value was calculated. The average values of the measurements are indicated by "Enduring" in the Tables.

In view of the mechanical strength of the cured product, adhesive properties to tooth structure, and durability, the preferred tensile strength after 1 day of immersion at 37°C (hereinafter, also referred to as "initial tensile strength") in the dental adhesive compositions of the present invention is 40 MPa or more, more preferably 45 MPa or more, even more preferably 50 MPa or more.

In view of the long-term mechanical strength of the cured product, adhesive properties to tooth structure, and durability, the preferred tensile strength after 7 days of immersion at 37°C (hereinafter, also referred to as "enduring tensile strength") in the dental adhesive compositions of the present invention is 25 MPa or more, more preferably 35 MPa or more, even more preferably 45 MPa or more.

Test Example 4: Measurement of Coating Thickness

The labial surfaces of bovine mandibular incisors were polished with #80 silicon carbide paper (manufactured by Nihon Kenshi Co., Ltd.) under running water to obtain samples with an exposed flat dentin surface. These samples then underwent additional polishing with #1000 silicon carbide paper (manufactured by Nihon Kenshi Co., Ltd.) under running water. After polishing, the surface was dried by removing remaining water by blowing air. A bonding area was defined by attaching an adhesive tape, approximately 150 μm thick and having a round hole with a 3 mm diameter, to the dried smooth surface.

The dental adhesive composition prepared in each Example and Comparative Example was applied into the round hole using an applicator brush (Fine <Silver>, manufactured by Kuraray Noritake Dental Inc.). After being allowed to stand for 10 seconds, the entire dental adhesive composition in the round hole was dried with a mild air blow at low to medium pressure until the liquid surface became stable. To ensure drying, the air blow was continued for at least 5 seconds. The dental adhesive composition was then cured into an adhesive layer by exposing it to light for 10 seconds using a dental visible-light irradiator (PenCure 2000, manufactured by J. Morita Corp.).

A dental filling composite resin (manufactured by Kuraray Noritake Dental Inc. under the trade name Clearfil® Majesty® ES Flow) was used to fill the surface of the cured product of the dental adhesive composition, and the surface was coated with a release film (polyester). Thereafter, a glass slide was placed on the release film, and pressed against the resin to flatten the applied surface of the composite resin. The composite resin was then cured by exposing it to light for 10 seconds through the release film, using the irradiator PenCure 2000. The sample was then immersed in distilled water inside a container, and left to stand in this state for 24 hours in a thermostatic chamber that had been set to 37°C.

After leaving the sample undisturbed for 24 hours, it was cut in half with a diamond cutter (ISOMET 1000 manufactured by Buchler, using a diamond cutting wheel) to expose the adhesive surface. The form of the sample was adjusted with #80 silicon carbide paper (manufactured by Nihon Kenshi Co., Ltd.) to allow for parallel placement of the observation surface, followed by precise polishing of the surface using a lapping film (manufactured by Sumitomo 3M Limited) in the sequence of #1200, #3000,and #8000. The resulting polished sample was secured to a sample stage using a double-sided carbon tape (HIS2166 manufactured by Hitachi High-Tech Fielding Corporation), and the coating thickness was measured using a scanning electron microscope (SU3500 manufactured by Hitachi High-Technologies Corporation) at 3,000× magnification in low vacuum mode.

Specifically, the adhesive layer within the 3 mm diameter round hole was measured for its thickness in a cross section of the sample cut in half to include the round hole and expose the adhesive surface. To reduce variation in the measured thickness of the adhesive layer, the outer 1 mm at both ends was excluded, and the average thickness of the adhesive layer was determined as the coating thickness based on three randomly selected points within the 1 mm center area, using software. Three samples were prepared, and the average was taken as the coating thickness (n = 3).

In view of the form of supporting teeth, the preferred coating thickness is 12 μm or less, more preferably 11 μm or less, even more preferably 10 μm or less.

Test Example 5: Viscosity Measurement with B-type Viscometer at 30°C

The dental adhesive composition prepared in each Example or Comparative Example was dispensed (0.7 mL) into a rotational viscometer (VISCOMETER TV-100 manufactured by Toki Sangyo Co., Ltd.). Stirring of the composition was initiated at a measurement temperature of 30°C with a rotation speed of 10 rpm. The viscosity at 30°C was measured 3 minutes after the start of stirring. For each dental adhesive composition, three measurement samples were prepared, and the average of the measured values was determined as the viscosity of the dental adhesive composition according to a B-type viscometer at 30°C.

[Table 1]

| Components (parts by mass) | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 |
|---|---|---|---|---|---|---|---|---|---|---|---|---|---|
| Monomer (A) having acidic group | MDP | 10 | 10 | 10 | 10 | 10 | 10 | 10 | 10 | 10 | 10 | | |
| | GDMP | | | | | | | | | | | 10 | |
| | 4-META | | | | | | | | | | | | 10 |
| Monomer (B-1) having three or more polymerizable groups per molecule | TMPMA | | | | 3 | | | | | | | | |
| | U4TH | | | | | 3 | | | 5 | | | | |
| | TAC4 | 3 | | | | | | | | | | | |
| | DTMP | | 3 | | | | 5 | 10 | | 3 | 3 | 3 | 3 |
| | Bis-4 | | | 3 | | | | | | | | | |
| Hydrophobic monomer (B-2) | Bis-GMA | 25 | 25 | 24 | 25 | 25 | 23 | 20 | 24 | 21 | 31 | 25 | 25 |
| | 3G | | | | | | | | | | | | |
| | MAEA | 12 | 12 | 12 | 12 | 12 | 12 | 10 | 12 | 11 | 12 | 12 | 12 |
| | UDMA | | | | | | | | | | | | |
| | NPG | | | | | | | | | | | | |
| Hydrophilic monomer (B-3) | DEAA | 10 | 10 | 10 | 10 | 10 | 10 | 10 | 10 | 12 | 10 | 10 | 10 |
| | HEMA | 10 | 10 | 10 | 10 | 10 | 10 | 10 | 10 | 10 | 10 | 10 | 10 |
| | #801 | | | | | | | | | | | | |
| Polymerization initiator (C) | CQ | 2 | 2 | 1.6 | 1.6 | 1.6 | 1.6 | 1.6 | 1.6 | 1.6 | 1.6 | 1.6 | 1.6 |
| | BAPO | 0.7 | 0.8 | 0.6 | 0.6 | 0.6 | 0.6 | 0.6 | 0.6 | 0.6 | 0.6 | 0.6 | 0.6 |
| | TMDPO | | | | | | | | | | | | |
| Water (D) | Purified water | 14 | 14 | 13 | 14 | 14 | 14 | 14 | 13 | 15 | 10 | 14 | 14 |
| Volatile organic solvent (E) | Ethanol | 16 | 16 | 18 | 16 | 16 | 16 | 16 | 16 | 18 | | 16 | 16 |
| | Acetone | | | | | | | | | | 14 | | |
| Filler (F) | R972 | 4 | 4 | 4 | 4 | 4 | 4 | 4 | 4 | 7 | 4 | 4 | 4 |
| Polymerization accelerator (G) | DABE | 2 | 2 | 2 | 2 | 2 | 2 | 2 | 2 | 1.6 | 2 | 2 | 2 |
| | DEPT | 0.7 | 0.7 | 0.7 | 0.7 | 0.7 | 0.7 | 0.7 | 0.7 | 0.6 | 0.7 | 0.7 | 0.7 |

(continued)

| Components (parts by mass) | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 |
|---|---|---|---|---|---|---|---|---|---|---|---|---|---|
| Others | BHT | 0.05 | 0.05 | 0.05 | 0.05 | 0.05 | 0.05 | 0.05 | 0.05 | 0.06 | 0.05 | 0.05 | 0.05 |
| | KBM-503 | | | | 0.5 | 0.5 | | | | | | | |
| | Sodium fluoride | 0.01 | | | | 0.01 | | | | | | | |

| | | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 |
|---|---|---|---|---|---|---|---|---|---|---|---|---|---|---|
| Water absorbency | % | Initial | 3.6 | 3.2 | 3.1 | 3.2 | 3.1 | 2.6 | 2.2 | 2.9 | 2.7 | 2.8 | 3.3 | 3.7 |
| | | Enduring | 7.3 | 6.0 | 6.6 | 6.8 | 6.6 | 6.2 | 5.8 | 6.4 | 6.2 | 6.8 | 7.4 | 7.9 |
| Shear bond strength (enamel) | MPa | Initial | 22 | 25 | 23 | 25 | 26 | 23 | 20 | 25 | 24 | 21 | 22 | 20 |
| | | Enduring | 16 | 18 | 17 | 18 | 19 | 18 | 16 | 18 | 19 | 15 | 16 | 16 |
| Shear bond strength (dentin) | MPa | Initial | 32 | 33 | 30 | 33 | 33 | 29 | 26 | 33 | 29 | 25 | 24 | 21 |
| | | Enduring | 28 | 28 | 26 | 28 | 28 | 24 | 22 | 28 | 25 | 20 | 21 | 16 |
| Tensile strength | MPa | Initial | 50 | 63 | 72 | 67 | 60 | 65 | 73 | 63 | 74 | 50 | 66 | 68 |
| | | Enduring | 42 | 56 | 60 | 54 | 48 | 48 | 60 | 40 | 62 | 42 | 52 | 52 |
| Coating thickness | µm | | 5 | 5 | 6 | 5 | 5 | 6 | 12 | 5 | 11 | 5 | 5 | 5 |
| Viscosity | cps | | 28 | 30 | 34 | 30 | 29 | 32 | 38 | 24 | 38 | 26 | 29 | 30 |

In the table, the monomer (B-1) having three or more polymerizable groups per molecule, hydrophobic monomer (B-2), and hydrophilic monomer (B-3) all refer to monomers with no acidic group.

[Table 2]

| Components (parts by mass) | | Ex. 13 | Ex. 14 | Ex. 15 | Ex. 16 | Ex. 17 | Ex. 18 | Ex. 19 | Ex. 20 | Ex. 21 |
|---|---|---|---|---|---|---|---|---|---|---|
| Monomer (A) having acidic group | MDP | 10 | 10 | 10 | 10 | 8 | 8 | 10 | 10 | 10 |
| | GDMP | | | | | | | | | |
| | 4-META | | | | | | | | | |
| Monomer (B-1) having three or more polymerizable groups per molecule | TMPMA | | | | | | | | | |
| | U4TH | | | | | | | | | |
| | TAC4 | | | | | | | | | |
| | DTMP | 3 | 3 | 3 | 3 | 0.3 | 15 | 3 | 3 | 28 |
| | Bis-4 | | | | | | | | | |
| Hydrophobic monomer (B-2) | Bis-GMA | | 25 | 25 | 25 | 35.7 | 17 | 25 | 25 | |
| | 3G | | 12 | | | | | | | |
| | MAEA | 12 | | | | 10 | 10 | 12 | | 12 |
| | UDMA | 25 | | | | | | | 12 | |
| | NPG | | | 12 | | | | | | |
| Hydrophilic monomer (B-3) | DEAA | 10 | 10 | 10 | 10 | 8 | 10 | 10 | | 10 |
| | HEMA | 10 | 10 | 10 | 10 | 8 | 10 | 10 | 10 | 10 |
| | #801 | | | | 12 | | | | 10 | |
| Polymerization initiator (C) | CQ | 2 | 2 | 2 | 2 | 2 | 2 | 2 | 2 | 2 |
| | BAPO | 0.7 | 0.7 | 0.7 | 0.7 | 0.7 | 0.7 | | 0.7 | 0.7 |
| | TMDPO | | | | | | | 2 | | |
| Water (D) | Purified water | 14 | 14 | 14 | 14 | 14 | 14 | 14 | 14 | 14 |
| Volatile organic solvent (E) | Ethanol | 16 | 16 | 16 | 16 | 16 | 16 | 16 | 16 | 16 |
| | Acetone | | | | | | | | | |
| Filler (F) | R972 | 4 | 4 | 4 | 4 | 4 | 4 | 4 | 4 | 4 |
| Polymerization accelerator (G) | DABE | 2 | 2 | 2 | 2 | 2 | 2 | 2 | 2 | 2 |
| | DEPT | 0.7 | 0.7 | 0.7 | 0.7 | 0.7 | 0.7 | 0.7 | 0.7 | 0.7 |

(continued)

| Components (parts by mass) | | | | | | | | | | | |
|---|---|---|---|---|---|---|---|---|---|---|---|
| Components (parts by mass) | | Ex. 13 | Ex. 14 | Ex. 15 | Ex. 16 | Ex. 17 | Ex. 18 | Ex. 19 | Ex. 20 | Ex. 21 | |
| Others | BHT | 0.05 | 0.05 | 0.05 | 0.05 | 0.05 | 0.05 | 0.05 | 0.05 | 0.05 | |
| | KBM-503 | | | | | | | | | | |
| | Sodium fluoride | | | | | | | | | 0.01 | |
| | | | Ex. 13 | Ex. 14 | Ex. 15 | Ex. 16 | Ex. 17 | Ex. 18 | Ex. 19 | Ex. 20 | Ex. 21 |
| Water absorbency | % | Initial | 2.9 | 3.7 | 2.6 | 3.8 | 3.2 | 2.4 | 3.5 | 3.7 | 2.5 |
| | | Enduring | 6.7 | 7.9 | 6.0 | 7.8 | 7 | 5.8 | 7.2 | 7.6 | 4.8 |
| Shear bond strength (enamel) | MPa | Initial | 18 | 25 | 22 | 23 | 27 | 22 | 25 | 20 | 16 |
| | | Enduring | 14 | 23 | 16 | 17 | 24 | 15 | 18 | 14 | 10 |
| Shear bond strength (dentin) | MPa | Initial | 29 | 26 | 24 | 28 | 26 | 24 | 30 | 23 | 20 |
| | | Enduring | 24 | 22 | 20 | 24 | 20 | 18 | 22 | 17 | 14 |
| Tensile strength | MPa | Initial | 64 | 53 | 42 | 62 | 50 | 75 | 55 | 58 | 80 |
| | | Enduring | 55 | 47 | 28 | 34 | 33 | 62 | 38 | 45 | 76 |
| Coating thickness | μm | | 5 | 5 | 5 | 7 | 11 | 10 | 5 | 11 | 5 |
| Viscosity | cps | | 25 | 27 | 27 | 31 | 37 | 35 | 30 | 37 | 25 |
| In the table, the monomer (B-1) having three or more polymerizable groups per molecule, hydrophobic monomer (B-2), and hydrophilic monomer (B-3) all refer to monomers with no acidic group. | | | | | | | | | | | |

[Table 3]

| Components (parts by mass) | | Com. Ex. 1 | Com. Ex. 2 | Com. Ex. 3 | Com. Ex. 4 | Com. Ex. 5 | Com. Ex. 6 | Com. Ex. 7 |
|---|---|---|---|---|---|---|---|---|
| Monomer (A) having acidic group | MDP | 10 | 10 | 10 | 15 | 10 | 10 | 10 |
| | GDMP | | | | | | | |
| | 4-META | | | | | | | |
| Monomer (B-1) having three or more polymer-izable groups per mo-lecule | TMPMA | | | | | | | |
| | U4TH | | | | 30 | 25 | | |
| | TAC4 | | | | | | 10 | 13 |
| | DTMP | | 10 | 3 | | | | |
| | Bis-4 | | | | | | | |
| Hydrophobic monomer (B-2) | Bis-GMA | 25 | 18 | 35 | | | 20 | 25 |
| | 3G | | | | | | | |
| | MAEA | 12 | 12 | 17 | | | 10 | 22 |
| | UDMA | | | | | | | |
| | NPG | | | | | | | |
| Hydrophilic monomer (B-3) | DEAA | 10 | 10 | 15 | | | 20 | |
| | HEMA | 10 | 10 | 15 | 30 | 30 | | |
| | #801 | | | | | | | |
| Polymerization initiator (C) | CQ | 2 | 2 | 2 | 1 | 1 | 2 | 2 |
| | BAPO | 0.7 | 0.7 | 0.7 | | | 0.5 | 0.7 |
| | TMDPO | | | | 2 | 2 | | |
| Water (D) | Purified water | 14 | 14 | 5 | 10 | 15 | 15 | 14 |
| Volatile organic solvent (E) | Ethanol | 16 | 16 | | 15 | | 15 | 16 |
| | Acetone | | | | | 20 | | |
| Filler (F) | R972 | 4 | | 4 | 5 | 5 | 7 | 4 |
| Polymerization accelerator (G) | DABE | 2 | 2 | 2 | 1 | 1 | 1 | 2 |
| | DEPT | 0.7 | 0.7 | 0.7 | | | 0.5 | 0.7 |
| Others | BHT | 0.05 | 0.05 | 0.05 | 0.05 | 0.05 | 0.05 | 0.05 |
| | KBM-503 | | | | | | | |
| | Sodium fluoride | | | | | | | |

| | | | Com. Ex. 1 | Com. Ex. 2 | Com. Ex. 3 | Com. Ex. 4 | Com. Ex. 5 | Com. Ex. 6 | Com. Ex. 7 |
|---|---|---|---|---|---|---|---|---|---|
| Water absorbency | % | Initial | 3.1 | 3.7 | 2.8 | 6.5 | 6.9 | 3.7 | 2.2 |
| | | Enduring | 7.2 | 7.9 | 7.0 | 8.4 | 8.2 | 7.3 | 3 |
| Shear bond strength (enamel) | MPa | Initial | 25 | 25 | 12 | 20 | 17 | 21 | 13 |
| | | Enduring | 20 | 23 | 6 | 16 | 13 | 15 | 9 |
| Shear bond strength (dentin) | MPa | Initial | 32 | 26 | 19 | 18 | 20 | 22 | 18 |
| | | Enduring | 30 | 22 | 15 | 14 | 16 | 17 | 10 |

(continued)

| | | | Com. Ex. 1 | Com. Ex. 2 | Com. Ex. 3 | Com. Ex. 4 | Com. Ex. 5 | Com. Ex. 6 | Com. Ex. 7 |
|---|---|---|---|---|---|---|---|---|---|
| Tensile strength | MPa | Initial | 33 | 26 | 60 | 30 | 28 | 56 | 83 |
| | | Enduring | 16 | 15 | 44 | 14 | 16 | 34 | 80 |
| Coating thickness | μm | | 5 | 5 | 40 | 5 | 5 | 14 | 18 |
| Viscosity | cps | | 29 | 27 | 820 | 29 | 25 | 45 | 70 |
| In the table, the monomer (B-1) having three or more polymerizable groups per molecule, hydrophobic monomer (B-2), and hydrophilic monomer (B-3) all refer to monomers with no acidic group. | | | | | | | | | |

As presented in Tables 1 and 2, the dental adhesive compositions of Examples yielded the following results.

The initial water absorbency was 3.8% or less. The enduring water absorbency was 7.9% or less.

For enamel, the initial shear bond strength was 16 MPa or more, and the enduring shear bond strength was 10 MPa or more.

For dentin, the initial shear bond strength was 20 MPa or more, and the enduring shear bond strength was 14 MPa or more.

In terms of mechanical strength, the initial tensile strength was 42 MPa or more, and the enduring tensile strength was 28 MPa.

The coating thickness was 12 μm or less. The viscosity was 38 cps or less.

In contrast, as shown in Table 3, the initial tensile strength was less than 34 MPa, and the enduring tensile strength was less than 17 MPa in Comparative Example 1, which lacked the monomer (B-1) having three or more polymerizable groups per molecule, and in Comparative Example 2, which lacked the filler (F).

In Comparative Example 3 with no volatile organic solvent (E), the viscosity was 820 cps, and the coating thickness was 40 μm.

In Comparative Examples 4 and 5 with no hydrophobic monomer (B-2), the initial water absorbency was 6.5% or more, and the initial tensile strength was 30 MPa or less.

In Comparative Example 6, the viscosity was high at 45 cps, and the coating had a thickness with a high value of 14 μm, failing to form a thin bond layer.

In Comparative Example 7 with no hydrophilic monomer (B-3), the initial bond strength and enduring bond strength to enamel were 13 MPa and 9 MPa, respectively. The coating thickness and viscosity were high, at 18 μm and 70 cps, respectively.

INDUSTRIAL APPLICABILITY

A dental adhesive composition of the present invention can be suitably used for filling applications, as well as for coating cavities and supporting teeth in dental treatment. In a dental adhesive composition of the present invention, the cured product exhibits high mechanical strength over an extended period even when the coating thickness is 12 μm or less (preferably less than 10 μm) in resin-coating applications, where a thin coating is required. That is, the cured product can retain its mechanical strength over a long period even when the cured layer becomes thinner than the intended thickness as a result of, for example, thickness variation occurring in the layer due to the extent of air blowing when the layer is formed by evaporating the solvent components (for example, by blowing air). Consequently, there is no need for exact procedures during use, making the dental adhesive composition particularly advantageous in resin-coating applications.

## Claims

**1.** A dental adhesive composition comprising a monomer (A) having an acidic group, a monomer (B) having no acidic group, a polymerization initiator (C), water (D), a volatile organic solvent (E), and a filler (F),

wherein the monomer (B) having no acidic group comprises a monomer (B-1) having three or more polymerizable groups per molecule, a hydrophobic monomer (B-2) having one or two polymerizable groups per molecule, and a hydrophilic monomer (B-3) having one or two polymerizable groups per molecule,
the dental adhesive composition has a viscosity of less than 40 cps according to a B-type viscometer at 30°C, and the dental adhesive composition has a water absorbency of 4.0% or less in its cured product.

2. The dental adhesive composition according to claim 1, wherein the monomer (B-1) having three or more polymerizable groups per molecule comprises a hydrophobic compound.

3. The dental adhesive composition according to claim 1 or 2, wherein the monomer (B-1) having three or more polymerizable groups per molecule comprises a compound having a urethane bond.

4. The dental adhesive composition according to any one of claims 1 to 3, wherein the monomer (B-1) having three or more polymerizable groups per molecule comprises an aliphatic compound.

5. The dental adhesive composition according to any one of claims 1 to 4, wherein the content of the monomer (B-1) having three or more polymerizable groups per molecule is 0.1 to 20 parts by mass in 100 parts by mass of all monomer components.

6. The dental adhesive composition according to any one of claims 1 to 5, wherein the polymerizable groups in the monomer (B-1) having three or more polymerizable groups per molecule are (meth)acryloyloxy groups.

7. The dental adhesive composition according to any one of claims 1 to 6, wherein the polymerization initiator (C) comprises at least one selected from the group consisting of a (bis)acylphosphine oxide, an $\alpha$-diketone, and a coumarin.

8. The dental adhesive composition according to any one of claims 1 to 7, wherein the volatile organic solvent (E) comprises an alcohol solvent.

9. The dental adhesive composition according to any one of claims 1 to 8, wherein at least one of the monomer (B-1) having three or more polymerizable groups per molecule, the hydrophobic monomer (B-2) having one or two polymerizable groups per molecule, and the hydrophilic monomer (B-3) having one or two polymerizable groups per molecule comprises a monomer having a (meth)acrylamide group.

10. The dental adhesive composition according to claim 9, wherein the hydrophobic monomer (B-2) having one or two polymerizable groups per molecule comprises a monomer having a (meth)acrylamide group.

11. The dental adhesive composition according to any one of claims 1 to 10, wherein the monomer (A) having an acidic group comprises a monomer having a phosphoric acid group and having one polymerizable group per molecule.

12. The dental adhesive composition according to claim 11, wherein the content of the monomer having a phosphoric acid group and having one polymerizable group per molecule is 90 parts or more by mass in total 100 parts by mass of the monomer (A) having an acidic group contained in the dental adhesive composition.

13. The dental adhesive composition according to any one of claims 1 to 12, wherein the monomer (B-1) having three or more polymerizable groups per molecule comprises a hydrophobic compound, and the polymerizable groups in the hydrophobic compound are all (meth)acryloyloxy groups.

14. A dental bonding material according to any one of claims 1 to 13.

15. A dental coating material according to any one of claims 1 to 13.

**INTERNATIONAL SEARCH REPORT**

International application No.
**PCT/JP2023/030400**

**A. CLASSIFICATION OF SUBJECT MATTER**

***A61K 6/20***(2020.01)i; ***A61K 6/30***(2020.01)i; ***A61K 6/62***(2020.01)i
FI: A61K6/30; A61K6/20; A61K6/62

According to International Patent Classification (IPC) or to both national classification and IPC

**B. FIELDS SEARCHED**

Minimum documentation searched (classification system followed by classification symbols)

A61K6/20; A61K6/30; A61K6/62

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2005-179282 A (KURARAY MEDICAL INC) 07 July 2005 (2005-07-07) claims, paragraphs [0013]-[0021], [0023], [0041], [0043], [0058]-[0083], table 2, examples | 1-15 |
| Y | JP 2021-054791 A (SHOFU INC) 08 April 2021 (2021-04-08) claims, paragraphs [0002], [0006], [0020], [0036], [0046], [0058], [0062]-[0064], table 1, examples | 1-15 |
| Y | JP 2021-054794 A (SHOFU INC) 08 April 2021 (2021-04-08) claims, paragraph [0045], examples | 1-15 |
| X | WO 2011/121966 A1 (KURARAY NORITAKE DENTAL INC.) 06 October 2011 (2011-10-06) claims, paragraphs [0031], [0033], [0100], examples | 1, 2, 4-8, 11-15 |
| A | JP 2008-260751 A (KURARAY MEDICAL INC) 30 October 2008 (2008-10-30) entire text, all drawings | 1-15 |
| A | JP 2001-288232 A (SUN MEDICAL CO LTD) 16 October 2001 (2001-10-16) entire text, all drawings | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 October 2023** | **07 November 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/030400**

**C. DOCUMENTS CONSIDERED TO BE RELEVANT**

| Category* | Citation of document, with indication, where appropriate, of the relevant passages | Relevant to claim No. |
|---|---|---|
| A | JP 2009-215254 A (TOKUYAMA DENTAL CORP) 24 September 2009 (2009-09-24) entire text, all drawings | 1-15 |

Form PCT/ISA/210 (second sheet) (January 2015)

**INTERNATIONAL SEARCH REPORT**
**Information on patent family members**

International application No.
**PCT/JP2023/030400**

| Patent document cited in search report | Publication date (day/month/year) | Patent family member(s) | Publication date (day/month/year) |
|---|---|---|---|
| JP 2005-179282 A | 07 July 2005 | (Family: none) | |
| JP 2021-054791 A | 08 April 2021 | US 2021/0000698 A1<br>claims, examples<br>US 2021/0000699 A1<br>US 2021/0007938 A1<br>EP 3733151 A1<br>EP 3733152 A1<br>EP 3733153 A1<br>KR 10-2020-0115255 A<br>KR 10-2020-0115256 A<br>CN 111743782 A<br>CN 111743783 A | |
| JP 2021-054794 A | 08 April 2021 | US 2021/0000698 A1<br>claims, examples<br>US 2021/0000699 A1<br>US 2021/0007938 A1<br>EP 3733151 A1<br>EP 3733152 A1<br>EP 3733153 A1<br>KR 10-2020-0115255 A<br>KR 10-2020-0115256 A<br>CN 111743782 A<br>CN 111743783 A | |
| WO 2011/121966 A1 | 06 October 2011 | US 2013/0012615 A1<br>claims, paragraphs [0030], [0107], examples<br>EP 2554155 A1<br>CN 102811695 A | |
| JP 2008-260751 A | 30 October 2008 | US 2010/0036075 A1<br>entire text, all drawings<br>WO 2008/087977 A1<br>EP 2108663 A1<br>CN 101589070 A | |
| JP 2001-288232 A | 16 October 2001 | (Family: none) | |
| JP 2009-215254 A | 24 September 2009 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008260751 A
- JP 2007223955 A
- JP 2005179282 A
- JP H093109 A
- JP H10245525 A
- WO 2008087977 A